# EUROPEAN PATENT SPECIFICATION

(11) **EP 1 996 255 B1**
(45) Date of publication and mention of the grant of the patent: **24.04.2013**
(21) Application number: 07752251.4
(22) Date of filing: 02.03.2007
(51) Int. Cl.: A61M 5/00

(54) **METHODS AND DEVICES FOR RETRIEVAL OF A MEDICAL AGENT FROM A PHYSIOLOGICAL EFFERENT FLUID COLLECTION SITE**
VERFAHREN UND VORRICHTUNG ZUR RÜCKGEWINNUNG EINES MEDIZINISCHEN WIRKSTOFFES AUS EINER PHYSIOLOGISCHEN EFFERENTEN FLÜSSIGKEITSSAMMELSTELLE
PROCÉDÉS ET DISPOSITIFS POUR EXTRAIRE UN AGENT MÉDICAL D'UN SITE DE COLLECTE DE FLUIDES EFFÉRENTS PHYSIOLOGIQUES

(30) Priority: 02.03.2006 US 778816 P; 13.07.2006 US 807297 P
(43) Date of publication of application: 03.12.2008
(73) Proprietor: Catharos Medical Systems, Inc., Campbell, CA 95008 (US)
(72) Inventor: FITZGERALD, Peter, J., Portola Valley, California 94028 (US); HASSAN, Ali, Mountain View, CA 94043 (US); COURTNEY, Brian, K., Toronto, Ontario M4V 2E9 (CA)
(74) Representative: Duxbury, Stephen
(86) International application number: PCT/US2007/005537
(87) International publication number: WO 2007/103279

(56) References cited:
- WO-A-86/03832
- WO-A-96/06654
- WO-A-03/045303
- GB-A- 2 350 794
- US-A- 3 616 272
- US-A- 4 103 685
- US-A- 4 652 255
- US-A- 4 792 689
- US-A- 4 939 468
- US-A- 5 113 358
- US-A- 5 649 905
- US-A1- 2002 143 286
- US-A1- 2004 254 523
- US-A1- 2005 004 476
- US-A1- 2005 100 937

## Description

### BACKGROUND

Administration of therapeutic or diagnostic agents to a subject is typically accomplished by either localized or systemic routes. With many types of agents, localized delivery methods are desirable. For example, medical compounds of interest may have desired diagnostic or therapeutic effects within the region into which they are introduced, but also exhibit toxic or other undesirable effects when they are allowed to circulate elsewhere. In certain cases, it is desirable to introduce a higher volume of a compound to the local region than can be tolerated by other body tissues if that volume were to ultimately cause the systemic concentration to exceed a safe threshold.

A common example of such a compound is radio-opaque dye. Iodinated forms of such a dye are used routinely during catheter-based interventional procedures such as coronary, renal, neurological and peripheral arteriography. The iodine component has a high absorption of x-rays and therefore provides a contrast medium for the radiological identification of vessels when introduced within an upstream artery. However, the use of such dyes is known to have potential toxic effects depending on the specific formulation, including direct injury to renal tubule cells, endothelial injury, bronchospasm, inflammatory reactions, pro-coagulation, anti-coagulation, vasodilation and thyrotoxicosis.

Other materials that may be introduced locally for desired effects but whose direct or other effects would be undesired elsewhere include vasoactive agents, cytotoxic agents, genetic vectors, apoptotic agents, anoxic agents (including saline), photodynamic agents, emboli-promoting particles or coils, antibodies, cytokines, immunologically targeted agents and hormones.

An important anatomic concept with respect to the vasculature and other conduits supplying and draining an organ is the principle that a tissue or organ and regions of the organ have a limited number of primary supply conduits and a limited number of draining conduits. Material introduced into the upstream side of the target tissue will typically be dispersed among the diverging arterioles and capillaries, which then reconverge into a collection of common venules and vein(s) downstream, e.g., in a physiological efferent fluid collection site. For example, the myocardium of the heart is fed by the right coronary, left anterior descending and left circumflex arteries. Each of these arteries enters a capillary network that eventually converges into the small and middle cardiac vein, anterior interventricular vein and posterior vein of the left ventricle. These veins are all tributaries of the coronary sinus, which may be viewed as a cardiovascular efferent fluid collection site. Material introduced into any of the aforementioned coronary arteries that travels through the capillary network will enter the coronary sinus providing an opportunity to collect it before it returns to the systemic circulation. In another example, the brain is fed by the carotid and vertebral arteries which enter a highly anastomotic network. Blood flow through the brain substantially drains to the systemic circulation via a network of sinuses that converge onto the internal jugular veins. In yet another example, each kidney is substantially supplied by a renal artery and drained by a renal vein. In yet another example, a tumor or metastatic lymph node may have a set of primary afferent (supply) conduits and a set of primary efferent (drainage) conduits. In yet another example, the lungs are supplied by a pulmonary artery and its branches, and are drained by the pulmonary veins and their tributaries into the left atrium.

### SUMMARY

WO86/03832 refers to an optical sensor for use in biomedical applications, and describes the closest prior art.

US2002/0143286 details a vacuum assisted wound closure device.

WO03/043303 discloses a device for preventing contamination of medical equipment due to effluent, such as stool, being expelled from a patient during an insufflation procedure.

Aspects of the invention include methods and devices for selectively removing an agent from a physiological efferent fluid collection site. In certain embodiments, an aspiration device is employed to selectively remove the target agent from the site, e.g., by removing fluid from the target site primarily when the target agent is at least predicted to be, e.g., anticipated and/or known to be, present in the site. Embodiments of the invention also include systems and kits for performing the subject methods. Embodiments of the invention find use in a variety of different applications, including the selective removal of both therapeutic and diagnostic agents from a variety of different physiological sites.

### BRIEF DESCRIPTION Of THE FIGURES

Figure 1 provides a block diagram of a representative system according to an embodiment of invention.
Figure 2 depicts a view a system according to another embodiment of the invention.
Figure 3 provides a view of components of the system of the embodiment shown in Figure 2.
Figures 4A to 4D provide a representation of the distal end of the aspiration/sensing catheter structure shown in the system depicted in Fig. 2.
Figure 5 provides a flow diagram of a protocol for using the system depicted in Fig. 2.
Figures 6A to 6C provide various views of the distal end of an aspiration catheter according to an embodiment of the invention.
Figures 7A to 7G provides a view of centering-anchor devices that may be present in embodiments of the invention.
Figures 8A to 8B and 9A to 9B provide depictions-of embodiments transmission sensors according to the present invention.
Figure 10 provides a depiction of a multi-detector sensor according to an embodiment of the invention.
Figure 11 provides a view of a deflective lens that may be present in embodiments of the invention.
Figure 12 provides a view of a graphical display of contrast agent detection that is produced according to an embodiment of the invention.
Figure 13 provides a view of an asymmetric balloon component of the embodiments of the invention.
Figure 14 provides a view of an elongation mechanism according to embodiments of the invention.
Figure 15 provides a depiction of a two different locations, A and B, that are efferent tributaries to an efferent fluid collection site, where detection may take place.
Figures 16A to 16C provide depictions of various fiber-optic tip configurations that may be present in certain embodiments of the invention.
Figures 17A and 17B provide a depiction of endovascular reflectance as occurs in certain embodiments of the invention.
Figure 18 provides a graph of results from an experiment which shows that capacitance based measurements may be employed to detect the presence of contrast agent in blood.

### DETAILED DESCRIPTION

Aspects of the invention include methods and devices for selectively removing an agent from a physiological efferent fluid collection site are provided. In certain embodiments, an aspiration device is-employed to selectively removed the target agent from the site, e.g., by removing fluid from the target site primarily when the target agent is at least predicted to be, e.g., anticipated and/or known to be, present in the site. Embodiments of the invention also include systems and kits for performing the subject methods. The subject invention finds use in a variety of different applications, including the selective removal of both therapeutic and diagnostic agents from a variety of different physiological sites.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope of the present invention. Any recited method can be carried out in the order of events recited or in any other order which is logically possible.

As summarized above, the present invention provides methods and devices, as well as systems and kits, for selectively removing an agent from a physiological efferent fluid collection site. In further describing the subject invention, the subject methods are reviewed first in greater detail, followed by a more in-depth description of representative embodiments of systems and devices for practicing the subject methods, as well as a review of various representative applications in which the subject invention finds use. Finally, a review of representative kits according to the subject invention is provided.

### METHODS

As summarized above, aspects of the invention include methods of selectively removing an agent from a subject (e.g., a patient), where embodiments of methods include removal of agent from an internal target site which is a region that is or is proximal to a physiological efferent fluid collection site. By physiological efferent fluid collection site is meant a site in a living entity such as an animal, where the site may be naturally occurring or artificially produced (such as by surgical technique), where fluid from two different sources or inputs combines or flows into a single location.

In certain embodiments, the animals in which the subject methods are employed are "mammals" or "mammalian," where these terms are used broadly to describe organisms which are within the class mammalia, including the orders carnivore (e.g., dogs and cats), rodentia (e.g., mice, guinea pigs, and rats), lagomorpha (e.g. rabbits) and primates (e.g., humans, chimpanzees, and monkeys). In certain embodiments, the subjects, e.g., patients, are humans.

In certain embodiments, the physiological efferent fluid collection site is a vascular efferent fluid collection site, where fluid from at least two different vessels joins into a single vessel. In certain embodiments, the vascular efferent fluid collection site is a cardiovascular fluid collection site, where fluid from at least two different veins joins into a single veinous structure. In certain embodiments, the cardiovascular efferent fluid collection site is the coronary sinus. In yet other embodiments, as indicated above, the efferent fluid collection site may be an artificially, e.g., surgically produced, fluid collection site, e.g., a non-naturally occurring fluid collection site produced by surgically joining two or more vessels together, etc.

In practicing embodiments of the methods, an agent (which in certain embodiments has been locally administered to a subject) is selectively removed from a target site, which target site is the physiological efferent fluid collection site or a region proximal thereto, e.g., downstream there from, where when the region is proximal thereto, in certain illustrative embodiments the target fluid removal site is no more than about 40 mm from the efferent fluid collection site, e.g., no more than about 15 mm from the efferent fluid collection site. By selectively removed is meant that the subject methods remove fluid from the target site in a manner that selectively or preferentially removes fluid from the site (and therefore from the body of the subject) that is at least predicted to include the agent, where in certain embodiments the removed fluid is not returned to the body, at least not without processing to remove the target agent present therein. In certain embodiments, because of the selective nature of fluid removal employed, only fluid that is at least predicted to include agent is removed, and during a given aspiration event, at least 50 %, such as at least 75%, including at least 90% or more of the fluid removed at any given time will include the agent to be removed. Depending on the particular protocol and device employed, as described in greater detail below, the fluid may be continuously collected at the fluid collection site but not removed from the body unless it is at least predicted to include agent, e.g., as occurs in those embodiments where fluid is collected at the fluid collection site but immediately shunted back to the subject if it is not at least predicted to include agent. By at least predicted is meant that the bulk or majority of the fluid removed from the site is fluid that is either anticipated to include the agent, e.g., fluid in which the presence of the agent is inferred, or fluid that is known to include the agent, e.g., fluid in which the presence of the agent is detected. Depending upon the particular embodiment of the invention being practiced; in selectively removing fluid from the target fluid collection site and subject, fluid may be removed from the site and subject for a period of time which commences prior to when agent is at least predicted to be in the site, and extend for a period of time after agent is at least predicted to be in the site. In such embodiments, the period of time during which fluid is collected before and/or after agent is at least predicted to be in the site is a fraction or portion of the total period of time during which fluid is removed, typically being less than 50%, such as less than 25% including less than 10-15% of the total time period during which fluid is removed.

In certain embodiments, the subject methods do not remove all fluid from a target and efferent fluid collection site, but just fluid that is at least predicted to include the target agent of interest. In other words, in practicing the subject methods, not all fluid from an efferent fluid-collection site present over a given period of time is removed, only fluid that is at least predicted to include the target agent of interest that is to be removed. Put another way, over a given period of time where fluid that does and does not include the target agent flows through the efferent fluid collection site and/or a target fluid collection site, only fluid that is at least predicted, e.g., is anticipated or known to include the agent, is removed from the site and subject, while fluid that does not likely include the target agent is preferentially not removed from the site and subject. In certain embodiments, the amount of fluid that is removed from a given site ranges from about 10mL to about 1000mL, such as from about 100mL to about 750mL and including from about 150mL to about 300mL.

Another aspect of certain embodiments of the subject methods is that not all of the agent that is administered prior to practice of the subject methods is removed from the subject. In other words, only-a portion of the administered agent is removed from the host or patient by the subject methods. By portion is meant that about 20% or more, such as about 50% or more and including about 70% or more of the administered agent is removed by the subject methods, where in certain embodiments, the portion removed is about 75% or more, about 80% or more, about 90% or more, or even greater. However, as not all of the agent is collected during practice of the subject methods, in certain embodiments about 1% or more of the originally administered agent remains in the subject, such as at about 5% or more or about 10% or more.

Agent is selectively removed from the target site, which may or may not be the efferent fluid collection site, according to the subject methods by removing, e.g., aspirating, fluid from the target site and subject, only when the target agent is at least predicted to be (or in certain embodiments known to be, e.g., by sensor) present in the target site, as described above. As such, when agent is at least predicted to be present in the target site, fluid is removed from the site and subject. Conversely, when agent is not predicted to be present in the site, fluid is not removed at least from the subject, and in certain embodiments not from the site. Accordingly, in certain embodiments, upon detection or anticipation of agent in the fluid collection site fluid is removed or aspirated from the site and subject, while when the target agent is not detected or anticipated to be present in the site, fluid is not removed from the site, with the exception of a short period of time before and/or after the time when agent is at least predicted to be in the target site, as described above.

In certain embodiments, fluid is selectively removed by actuating a fluid removal element, e.g., aspiration device, such as the devices described below, a defined period of time following administration of the agent to the subject, e.g., an absolute preset period of time, a period of time as defined by a physiological metric, e.g., heart beat, etc.

In certain embodiments, the methods include a step of detecting the presence of target agent at a target sensing site (e.g., the efferent fluid collection site, a site proximal thereto, e.g., a site upstream, etc.) and then removing fluid, and agent present therein, from the site in response to detection of the presence of target agent in the site. Typically, when agent is no longer detected in the efferent fluid collection site, the methods stop removing fluid from the site. Thus, fluid is only removed from the efferent fluid collection site and subject over a time period that substantially overlaps the period in which the target agent is present in the efferent fluid collection site.

In practicing these embodiments of the subject methods, the agent may be detected in the fluid collection site using a number of different protocols. In certain embodiments, agent is visually detected by a skilled operator, who then removes fluid in response to visualizing agent, e.g., according to the protocols described below, present in the fluid collection site. In yet other embodiments, agent detection devices that are operatively connected to a fluid removal device are employed, where a signal from the detector that agent is present in the fluid collection site automatically actuates a fluid removal device, e.g., aspiration unit. Representative embodiments of devices that may be employed in such embodiments are described in greater detail below.

To aid in the detection of the agent, in certain embodiments the agent will be one that is labeled with a detectable label, e.g., agent that has-been labeled with a detectable label prior to its introduction into the patient. The agent may be directly labeled with the detectable label, or associated with a detectable label such that the agent is indirectly detectable in that detection of the label also indicates the presence of agent which is presumed or inferred to be within the vicinity of the label. The nature of the label may vary, and may be a radio label, fluorescent label, chromogenic label (e.g., that has a pigment detectable in the optically visible spectrum), etc.

In certain embodiments, the pressure of the target site and/or efferent fluid collection site (which may or may not be the same locations, as described above) and or the tributaries thereof, including a subset of the tributaries thereof, may be modulated, e.g., reduced, in order to achieve the desired collection of agent from the subject. The manner in which the pressure may be modulated may vary depending on the particular device employed and manner in which it is implemented, where representative devices and protocols capable of pressure modulation of the target/efferent fluid collection site are described in greater detail below. By modulating the pressure in this manner, one can reduce the pressure within the collection site sufficiently to improve the efficacy of removing the desired agent without causing collapse of the tributaries of the efferent fluid collection site, resulting in a better or more favorable outcome of the method.

In certain embodiments, devices that include a shunting element, be it a passive or active shunting element, are employed in a manner that modulates the pressure of the target site and/or efferent fluid collection site, as desired. Alternatively and/or in addition thereto, one can use a pressure sensor within the fluid collection site. The output from such a sensor may be used to optimize the maintenance of the pressure in the collection site so that it is reduced sufficiently in order to increase the likelihood of higher flow to that region from those tributaries that have alternative paths, without causing the collapse of such tributaries.

In certain embodiments, an extension of an aspiration lumen of the device employed is extended selectively into one or more tributaries in order to prevent their collapse during aspiration and to extend the volume from which fluid is aspirated. Alternatively, rather than using a lumen-to structurally support the tributaries, a temporary or permanent stent could be introduced to those tributaries prior to aspiration. As a specific example, in certain embodiments the small cardiac vein is stented for such purposes, or a branch of the aspiration lumen from the coronary sinus is extended through the small cardiac vein and/or middle cardiac vein for such purposes.

In certain embodiments, a specific pattern of aspiration rates that compensates for the delay time between the detection of the desired agent and the activation of the aspiration mechanism is employed. For example, in certain embodiments, there will be a small but finite delay in time between when the agent enters the fluid collection site and when the aspiration mechanism begins to aspirate fluid from the site. During this time delay, some of the fluid containing the agent may have already passed the region from which aspiration normally occurs at the distal portion of the aspiration lumen, thus potentially reducing the efficacy of retrieving the agent. However, by having a higher rate of aspiration for the early portion of the period in which aspiration occurs, as compared to a rate that more closely resembles the normal physiologic rate of flow within the collection site, e.g., where the higher aspiration rate is about 2-fold greater, such as about 5-fold or 10-fold greater, one can cause that fluid which has already passed the region from aspiration to change direction and return to the aspiration ports. Once this initial period of a higher rate of aspiration has expired, the aspiration rate could then occur at a lower rate which more closely approximates the normal physiologic rate of flow within the collection site, as desired.

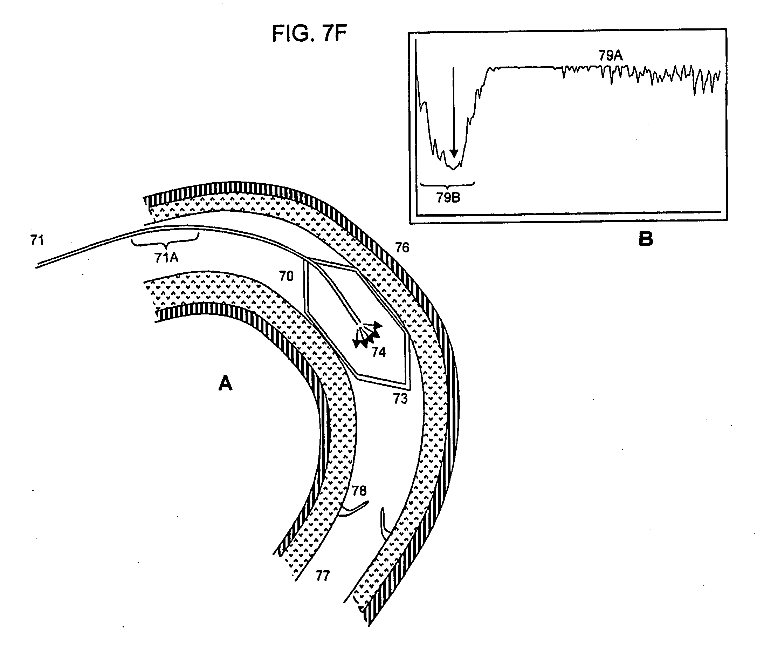

In certain embodiments, the agent to be removed may be a hyperemic-agent, by which is meant that the presence of the agent in the vasculature is associated with a hyperemic state which can be detected/determined/assessed in the fluid collection site, e.g., by causing vasodilation in an efferent fluid collection site. In such embodiments, one may employ a variable aspiration rate which is adapted to the hyperemic state of the efferent fluid collection site. For example, where a hyperemia inducing contrast agent is to be removed from the efferent fluid collection site (e.g., coronary sinus), the aspiration rate may increase over time, e.g., to accommodate hyperemia associated increases in flow in the target vessel. As such during an aspiration event the rate may increase from a first rate to a second rate, where the second rate is larger than the first rate. The rate may be adapted to detection of a signal produced by a hyperemic agent, see e.g., panel B in Fig. 7F.

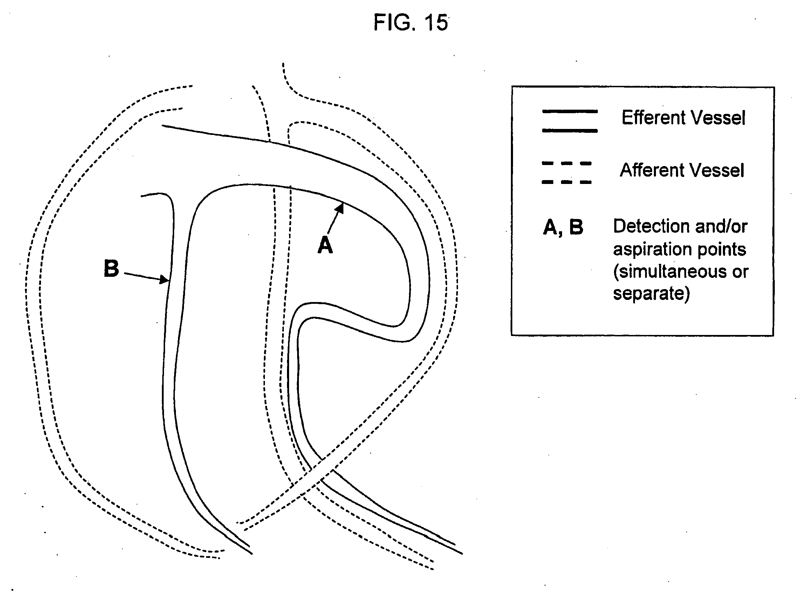

In some embodiments, more than one kind of detector is employed to determine the aspiration parameters and time period. For example, in order to ensure that the leading edge of the agent is successfully aspirated, the activation of the aspiration mechanism may be activated by a counter that counts a conservative, pre-selected number of QRS complexes on an EKG after the beginning of injection of the agent. In certain embodiments, the trigger to deactivate the aspiration mechanism may be derived from an optical sensor that can recognize when there is no longer any more agent within the fluid being aspirated. Alternatively, the deactivation signal may be generated by a processor which obtains data, either raw data or processed in some way, from the sensor, which may or may not be a sensor that is different from the detection sensor, and employs an algorithm, e.g., that uses one or more decision rules, to determine whether aspiration should continue or stop. Such deactivation systems may be employed where a more complex analysis is required, e.g., where the target agent to be removed is a hyperemic agent. In certain embodiments, inputs from more than one detector can be used in direct combination with each other to determine the aspiration parameters. In certain embodiments, inputs can be obtained from two or more different locations, e.g., two or more tributaries of the target efferent fluid collection site. FIG. 15 provides, a depiction of a two different locations, A and B, that are efferent tributaries to an efferent fluid collection site, where detection may take place, e.g., where detector components may be positioned. In certain embodiments, due to cardiac motion in the region of a fiber optic based sensor, and/or variations in the rate of flow of the fluid in the region of the sensor, the signal produced may vary in a pattern that is reflective of the cardiac cycle, regardless of whether or not the agent to be detected is present, thus producing a noisy signal. In such.a case, the fidelity of the sensor may be augmented by using a filtering algorithm that uses the input from an EKG signal to filter the signal produced by the optical detector. By compensating for changes to the output of the optical detector that are due to the cardiac cycle, it may be easier to more accurately characterize the concentration of the agent to be removed in the region of the detector. Any of the detectors mentioned below may be suitably used in combination with each other to further optimize the detection process and/or the efficacy of the aspiration controller.

Practice of the subject methods results in selective removal of an agent from a fluid collection site and subject, where the amount of agent removed is, in certain embodiments, a substantial portion of (but not all of in certain embodiments) the agent that is present in the subject, as described above.

In certain embodiments, the fluid that is removed from the subject may be treated extracorporally, e.g., to remove or neutralize the agent, and then reintroduced into the subject, e.g., where it is desired to minimize the ultimate or final volume of fluid, e.g., blood, that is removed from the subject in a given procedure. For example, where the fluid removed from the subject is blood, the removed blood may be processed with a blood filtering device to remove the agent from the blood, and the processed blood, or at least a component thereof (such as red blood cells) be returned to the patient. Examples of representative fluid, e.g., blood, processing devices include, but are not limited to: the Cell Salver® device (available from Haemonetics); autoLog (available from Medtronic); and the like.

As such, the subject methods may-include a step of transferring the harvested fluid into a recirculating system to be reintroduced into the body (as described in U.S. Patent No. 5,925,016, ). The recirculating system may incorporate mechanisms to separate the substantially undesirable components from the substantially desirable components. Such a system may incorporate a filter, a centrifugal separator, flow cytometry or other similar apparatuses. The aspiration mechanism may incorporate fluid characterization elements by which aspirated fluid may be characterized, either quantitatively or qualitatively.

Accordingly, in certain embodiments the subject may be one in which it is desired to keep blood loss at a minimum, e.g., the patient may suffer from coronary artery disease, chronic anemia, etc. Extracorporeal processing and subsequent reinfusion of the treated fluid allows for the reintroduction of the desirable components as an autologous transfusion. Centrifugal mechanisms, filter-based systems, dialysis membranes and cell-washing mechanisms are examples of some functional components that can be employed for this purpose.

In certain embodiments, the methods include delivery of an agent to a patient in manner which maximizes agent removal from the patient. For example, during contrast angiography, a volume of injected contrast may leak from an injection catheter into a blood vessel not targeted by angiographic procedure, i.e., into a non-target vessel. In such instances, contrast is thus routed away from target tissue/organ, and would therefore be an un-capturable volume of agent. In the case of contrast angiography, this is caused most frequently by too rapid injections, at high pressures (exceeding the local pressure in target vessel), and/or a mis-match between injection catheter size and target vessel size. In such situations, the delivery pressure may be modulated to minimize leakage of agent into unwanted body sites. For example, a pressure sensor installed into the injection pathway indicating the injection pressure of contrast agent, and providing feedback to the operator to adjust injection pressure to desirable values may be employed. Alternatively or in addition, the local pressure in target injection vessel can be sensed to provide an optimal range of injection pressure prior to injection. Alternatively or in addition, the local pressure in target injection vessel can be modified (e.g. lowered) to produce favorable conditions encouraging contrast to flow into target injection vessel. As such, pressure of delivery and/or at the target site may be modulated to minimize wayward agent. Alternatively or in addition, a system and device for contrast detection and removal can in associated with contrast injection catheter. Such systems/devices can detect wayward contrast around the injection catheter, followed by activating aspiration of fluid surrounding the injection catheter including the wayward contrast.

Where desired, components of the systems, e.g., the aspiration catheter and/or sensor catheter, can be equipped with mechanisms for steering the distal end of the devices, e.g., tip, during navigation of the target vasculature. Such mechanisms include (but are not limited to); wire-based, electronic, hydraulic, magnetic, and other means of steering.

The methods may be carried out using any convenient system/device, where in certain embodiments, catheter based systems/devices are of interest. Representative systems/devices for use in practicing the subject invention are reviewed in greater detail in the following section.

### DEVICES AND SYSTEMS

Also provided by the subject invention are devices and systems for selectively removing an agent from an efferent fluid collection site according to the methods described above. Aspects of the invention include devices specifically designed to selectively remove fluid from the efferent fluid collection site, where in certain embodiments of particular interest, as described in greater detail below, the devices are characterized by being non-occlusive, in that they lack an occlusive element, specifically at their distal end. By non-occlusive is meant that, at least while fluid and agent is not being removed from the collection site and subject, fluid enters and leaves the device while not passing outside of the subject, i.e., while remaining intracorporeal. Thus, in certain embodiments, the device is non-occlusive because at no time during its operation does it assume a configuration where the vessel in which it is placed is occluded. In yet other embodiments, the device may be configured so that it collects all fluid at a particular fluid collection site, but then provides for exit of the collected fluid out of the device (when agent is not to be removed from the subject) at a location such that the fluid always remains in the body (e.g., at the distal end of the device), and does not pass out of the body prior to its return to the body, i.e., the harvested fluid is always intracorporeal. Depending on the particular device being employed, the fluid may be returned to the body at essentially the fluid collection site, or at a region downstream from the fluid collection site. In these latter embodiments, while the device may be configured to collect all fluid from the fluid collection site, it is non-occlusive for purposes of the present invention because the fluid can be selectively returned to the subject without passing outside of the body, so as to practice the subject methods in which only fluid that is at least suspected of containing the target agent is removed from the subject, as developed more fully above. It should be noted that in these latter embodiments, when fluid is at least suspected of containing agent is removed from the body, the device may assume a configuration such that essentially all fluid is collected and removed from the fluid collection site.

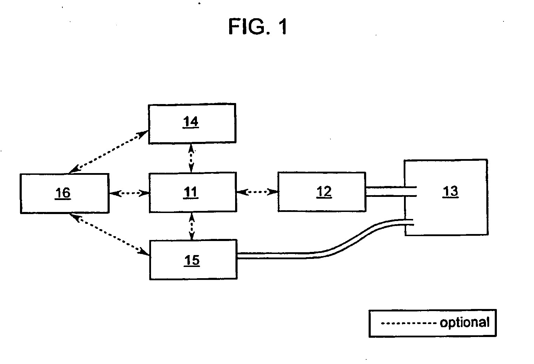

The subject systems are collections or combinations of disparate elements that include the subject devices, such as an aspiration element and controller thereof, as well as other components employed in the subject methods, e:g., one or more agent detectors, data recorders/displayers, delivery systems, and the like. See Figure 1 for a diagram of a system according to the subject invention.

In using the below described embodiments of the devices for practicing the aspects of the methods, the aspiration element, e.g., lumen(s), is placed in the at least one region at which fluid from the introduction site(s) of the agent to be removed ultimately converges (i.e., a physiological efferent fluid collection site), such as the coronary sinus. The aspiration mechanism communicates to the proximal end of each aspiration lumen and is able to cause the removal of fluid from the region at the distal end of the aspiration lumen. The aspiration controller, when present, contains mechanisms to control the degree to which the aspiration mechanism is activated over time.

Optionally, the invention may incorporate the use of a detector that provides one or more signals to the controller that can then be used to determine the timing and degree to which the aspiration mechanism is activated. Optionally, the invention may incorporate the use of one or more signals from the injection / delivery system as inputs to the controller. The controller may include a timer, or a device able to count EKG cycles to determine the degree of activation of the aspiration mechanism over time. Optionally, the invention may incorporate the use of a recording device and/or interactive display to either log and/or display the activity of the system during a procedure, or to change parameters that govern the operation of the system's components.

The subject devices and systems are now described in greater detail separately.

### Aspiration Element

In certain embodiments of the subject invention, the devices at least include: an aspiration element; which element may include: (a) at least one aspiration lumen; and (b) an aspiration mechanism; where in certain embodiments the aspiration element may further include an aspiration controller. Each of these elements, both constant and optional, are now reviewed in greater detail.

### Aspiration Lumen

In the subject devices, one or more aspiration lumens are provided, where the aspiration lumen(s) is constructed or configured in such a manner to be introduced into the target collection site, e.g., efferent fluid collection site or a site proximal thereto, e.g., via a body conduit such as the venous vasculature or a surgically produced access point, so that the distal end can be positioned in the target site for collection of the introduced medium. In certain embodiments where the target efferent fluid collection site is a cardiovascular efferent fluid collection site, e.g., as in the case of retrieving compound-laden fluid from the coronary sinus, there may be a catheter with a length appropriate for introduction through either a brachial, jugular or femoral access site to be advanced to the coronary sinus, such as over a guidewire or similar element, for percutaneous delivery. In these embodiments, the aspiration lumen is a catheter device, having dimensions sufficient to be introduced into the efferent fluid collection site via a vascular, e.g., veinous route, where such dimensions are known and readily determined by those of skill in the art.

In certain embodiments, the aspiration lumen has more than one diameter along its length. For example, in order to more easily enter or approach a collection site, the distal portion of the aspiration catheter is of a first diameter such that the distal portion fits within the geometric constraints of the anatomy of the collection site. In order to reduce the resistance to flow along the entire length of the aspiration lumen, the aspiration lumen has a second, larger diameter for one or more proximal segments of the aspiration lumen. In some cases where a high degree of flow may be required in order to successfully aspirate all the fluid that enters the collection site, such a configuration helps to reduce the total resistance of the lumen, which is proportional to the fourth power of the radius and is thus very sensitive to lumen diameter.

As indicated above, the aspiration lumen is, in certain embodiments, specifically constructed to be non-occluding. As such, the aspiration lumen of these embodiments does not include an occlusive element, e.g., a balloon or other element designed to occlude a vessel or conduit. As such, the subject devices of these particular embodiments are occlusive element free devices.

In certain embodiments, the aspiration lumen may include an optically transparent distal region (e.g., the tip may be constructed from an optically transparent material). In these embodiments, the optically transparent region of the distal end may be fabricated from a material that is transparent to light having a wavelength of from about 200nm to about 990nm. The optically transparent region may have a portion that is commensurate with the distal end of the aspiration lumen, or be located some short distance from the distal end of the aspiration lumen, e.g., where the optically transparent region is an optically transparent window. In certain of these embodiments where an optically transparent region is present at the distal end of the aspiration lumen, an optical sensor (e.g., as described below) is positioned inside the lumen at the distal end. Provision of an optically transparent region at the distal end of the lumen in such embodiments allows the sensor to detect an agent, e.g., contrast, that may be entering the fluid collection site at a point that is on the side of lumen, e.g., from the coronary ostia, and not upstream of the lumen.

In certain embodiments, the aspiration lumen may include a physiological parameter sensor located at its distal end. Types of physiological parameter sensors that may be located at the distal end of the lumen include, but are not limited to: doppler sensors, ultrasound sensors, optical sensors, pressure sensors, pH sensors, oxygen sensors, carbon dioxide sensors, other energy detection sensors, etc. Locating sensors at the distal end of the aspiration can provide a number of benefits. For example, positioning a pressure sensor at the distal end of the aspiration lumen provides the ability to monitor pressure variations at the fluid collection site and tailor the aspiration profile. In addition, a pressure sensor can be used to detect a rapid drop in pressure at the aspiration site should a collapse occur, such that remedial intervention can be rapidly performed. In these embodiments, the sensors can be separate structures associated with the distal end of the aspiration lumen or integrated into a portion or region of the aspiration lumen. In certain embodiments, an optical sensor as described elsewhere may be integrated into a region of the aspiration lumen, e.g., at a position in the distal region of the aspiration lumen.

In certain embodiments, the distal end of the aspiration catheter has a configuration that assists in positioning it at a desired location of an efferent fluid collection site. For example, in certain embodiments the distal end of the catheter has a loop-configuration, which loop-configuration, such as an "alpha" loop (e.g., where the distal tip assumes an "α" shape), helps to secure the distal end of the aspiration catheter at the level of the ostium of the coronary sinus.

### Aspiration Mechanism

In the subject devices, each aspiration lumen is operatively connected to at least one aspiration mechanism. There may be more than one aspiration lumen connected to each aspiration mechanism. The aspiration mechanism serves the purpose of withdrawing fluid from the target region via the aspiration lumens. The aspiration mechanism may, in certain embodiments, then dispose of the fluid, transfer the fluid into a re-circulating system to be reintroduced into the body (as described in U.S. Patent No.5,925,016, ), or simply store the fluid in a reservoir, as desired. The re-circulating system may incorporate mechanisms to separate the substantially undesirable components from the substantially desirable components. Such a system may incorporate a filter; a centrifugal separator; flow cytometry or other similar apparatuses. The aspiration mechanism may incorporate fluid characterization elements by which aspirated fluid may be characterized, either quantitatively or qualitatively.

In certain embodiments, the aspiration mechanism is one that provides for aspiration rates of between about 10 to about 1000ml/min, including from about 10 to about 700ml/min, such as from about 10 to about 500ml/min.

One embodiment of the aspiration mechanism is a suitably-sized syringe in fluid communication with the aspiration lumen. Upon activation by the aspiration controller, the plunger of the syringe is retracted, causing the aspiration of fluid. Any of several mechanisms can be used to provide the motor force necessary to retract the syringe. A rotary motor attached to a threaded bar can be used to cause a pullback motion by coupling the plunger to a component similar to a mechanical nut or other thread-receiving implement that attaches to the threaded bar. A variant of such an embodiment is to attach the thread-receiving implement to the motor and have the threaded component on the plunger. Alternatively, a rotary motor can wind a cable attached to the plunger, or a rack and pinion system may be employed. Alternatively, the motor force can come from a preloaded spring that has sufficient energy stored within it to cause the withdrawal of the plunger. Alternatively, the motor force may come from a compressed gas compartment, or a vacuum compartment.

An alternative embodiment is to have a compartment within which a vacuum exists and the withdrawal of substance occurs by allowing fluid communication between the vacuum compartment and the aspiration lumen. This vacuum element would be similar to the principle used for phlebotomy that is incorporated in the Vacutainer® system.

An alternative embodiment is to use a roller pump, whereby rollers external to the aspiration lumen near the proximal end of the aspiration lumen compress a soft portion of the tubing, and push the contents of the lumen towards the proximal end.

An alternative embodiment is to have an aspiration lumen whereby the proximal end of the aspiration lumen is in fluid communication with the ambient environment or a container whose internal pressure is equal to that of the ambient environment so that the pressure differential between the venous circulation and the ambient environment provides a significant portion of the necessary mechanical impetus to cause aspiration.

Yet another alternative embodiment is to have an aspiration lumen whereby the proximal end of the aspiration lumen is placed at a lower altitude than the distal end of the aspiration lumen, so that the difference in potential energy of fluid at each of these locations causes fluid to flow primarily by gravitational forces out the proximal end.

Depending on performance requirements for the particular application at hand, each of these mechanisms may either have strict binary activation (on or off), or their degree of activation may be controllable. Actuators for controlling the extent of activity may include valves, braking mechanisms, electronic controllers, amplifiers and other common mechanisms.

### Aspiration Controller

As indicated above, in certain embodiments the aspiration element further includes an aspiration controller. In certain embodiments, however, an aspiration controller is not present, e.g., in those embodiments where-the aspiration mechanism is a syringe that is operated manually by a health care professional.

When present, the aspiration controller is an element that actuates the aspiration element in response to an input signal, e.g., where the input signal may be provided by the operator performing the method or a detector element, as described below. The aspiration controller may actuate the aspiration element in a simple on/off manner, or may actuate the aspiration element in a more complex manner, e.g., to varying degrees over time, such that the aspiration controller may provide a way for controlling the degree to which the aspiration mechanism is activated over time.

The aspiration controller accepts one or more inputs. Such inputs can include manual inputs, e.g., from a health care professional performing the method, or signals from one or more detectors or instruments. As such, in certain embodiments, the subject devices are employed with one or more detector components, where the detector components may or may not be integral to the devices, i.e., may or may not be part of the devices.

In certain embodiments of the subject methods, two goals of the process of selectively retrieving an agent after it has been introduced are considered in the design and/or operation of the current invention. The first goal is to retrieve a high percentage of the introduced material, while the second goal is to remove as little of the native fluid (e.g., blood) as possible. In certain embodiments, these goals may be in conflict with each other. For example, the retrieval of a higher percentage of the introduced material may most easily be obtained by aspirating a higher volume of native fluid, while the removal of a lower volume of native fluid (e.g., blood) may most easily be obtained by aspirating a lesser volume of the introduced material. Therefore, it may be desirable to incorporate into the controller a method to vary the concentration threshold at which the aspiration mechanism is activated. A lower threshold would increase the percentage of agent retrieved, while a higher threshold would minimize the amount of native fluid retrieved.

The threshold of agent concentration for activation of aspiration may be different than the threshold of agent concentration for deactivation of aspiration. Alternatively, the rate of aspiration may be a more continuous function of the agent concentration. For example, higher agent concentrations may indicate to the controller that the rate of aspiration may be increased. Alternatively, the rate of aspiration may be a function of both agent concentration and time.

Several other parameters can be controlled to optimize the goals of retrieval and efficiency, depending on the particular protocol being performed. For example, the injection rate and/or aspiration rate may be adjusted to produce an optimal retrieval.

If the aspiration rate were to be less than the physiologically relevant flow rate through the targeted region, then a certain fraction of the introduced agent would flow past the distal end of the aspiration lumen and not be retrieved. Conversely, if the aspiration rate were to be greater than the physiologically relevant flow rate through the targeted region, an excess amount of native fluid may be aspirated, some of which may arrive to the aspiration lumen by traveling in a retrograde manner. Matching the aspiration rate with the physiological flow rate through the targeted region of retrieval provides, in certain embodiments, a desirable optimum solution. Flow rates may be determined and employed to assist in selecting the aspiration rate. For example, a sensor for detecting a flow rate at the distal end of the aspiration lumen may assist in achieving this optimization. Flow rates may also be determined through pressure measurement in directly or indirectly connected compartments (in fluid communication with the target compartment); e.g. any pressure sensor in the right atrium (RA) or one of the cava veins (IVC and SVC) can be employed to provide accurate information about the endovascular pressure (and thus flow rates if cross-sectional area is known) in the terminal portions of the coronary sinus (CS), since all (RA, IVC, SVC, and CS) are connected to the same fluid system.

Similarly, at the injection site, if more agent is introduced than can be immediately accepted at the injection site for antegrade flow, that agent may get diverted to the systemic circulation and can thus not be collected efficiently at the targeted collection region. This situation may be seen to occur under fluoroscopy as angiographic dye is injected near coronary ostia, wherein the excess dye flows back into the aorta and is essentially wasted for diagnostic purposes, while still increasing the system concentration of the agent. However, if less agent than can be immediately accepted for antegrade flow is injected into the injection site, the agent will be diluted with native fluid. This early dilution will worsen the efficiency of retrieval of the agent at the target site, since more native fluid will have to be aspirated in order to retrieve a fixed targeted volume of the agent. A sensor for detecting the flow rate at the site of injection is employed, in certain embodiments, to achieve this optimization.

Furthermore, the controller may incorporate a dynamic component in its control algorithm (i.e., it may be an adaptive controller) whereby the percentage of agent retrieved during a cycle of injection/aspiration is sensed, and the controller adjusts parameters for the cycle, such as, but not limited to, a concentration threshold for aspiration and/or the injection rate and/or aspiration rate and/or the duration of aspiration. These adjustments can be made iteratively over consecutive cycles in an attempt to optimize the parameters of injection and aspiration for subsequent cycles.

### Detector Components

In certain embodiments, the system includes a detector (i.e., sensor) component, e.g., for detecting the agent of interest (or a proxy therefore). The agent of interest may be detected using a number of different approaches. In certain embodiments, properties of the agent itself are detected. For example, specific binding of the agent may be employed, e.g., using a binding event sensor; optical/photometric approaches for detecting the agent, e.g., reflectance, transmission, evanescence, etc., may be employed; physical, e:g., viscosity, changes caused by the agent may be employed; electrical, e.g., conductivity, changes caused by the agent may be employed; radioactive, e.g., radiosorbance, approaches may be employed; fluorescence changes caused by the agent may be employed; acoustic, e.g., ultrasonic: echogenicity, scattering, etc. changes caused by the agent may be employed, etc.

In certain embodiments changes in the fluid caused by the presence of the agent are employed to detect the presence of the agent. Changes of interest in a given fluid include, but are not limited to:changes in number of blood cells per volume; changes in optical properties; changes in chemical properties; changes in physical properties (density, hematocrit, viscosity); changes in hemodynamic properties (velocity); changes in overall imaging properties of blood- (ultrasonic, radioactive, radiosorbent, fluorescent, etc.

A number of different detector components may be employed with the subject devices. Possible detectors or instruments that would be generally external to the body include EKG leads, fluoroscopic images, an automated injection system and/or a manually triggered signal from a technician. The controller could then execute a profile of aspiration over time based on the time from injection or manual triggering. Such a profile may be timed over a number of cardiac cycles or over conventional time. The pattern and/or density of pixels in the fluoroscopic images could also be used to recognize the injection and/or migration of material that produces imaging contrast.

In yet other embodiments, detectors of interest include fiber-optic based sensors, temperature sensors, acoustic sensors, pH detectors, capacitance-based detectors, fluid velocity detectors, conductivity detectors and detectors able to detect changes in ferro-electromagnetism or magnetic susceptibility (see e.g., Blood, 1 January 2003, Vol. 101, No. 1, pp. 15-19).

When employed, sensor location during a given operation may be recorded and stored into a memory, e.g., of the system, as desired.

### EKG Inputs

In certain embodiments, signals from EKG electrodes are used to provide a physiologically based timer wherein the controller incorporates a delay between the time of injection of material that is based in part or in entirety according to the number of cardiac cycles that have elapsed rather than using absolute time measured in seconds. The two measures of time are combined, in certain embodiments, to develop an algorithm to trigger the pattern of aspiration relative to the time of injection. For example, the algorithm may cause aspiration to begin based after either a preset number of cycles (e.g. 3.5 cardiac cycles) has elapsed, or an absolute amount of time (e.g. 8 seconds) has elapsed, whichever comes first. The use of cardiac cycles is of interest in many embodiments because it is related to the degree of blood flow in most organs. The heart rate may also be used to determine the peak rate of aspiration and the time-course over which the aspiration is active. A more rapid heart rate may indicate that aspiration could be optimized by having the aspiration occur more rapidly and/or over a shorter period of time.

The EKG leads may be either externally placed on the skin, as in conventional EKG and/or may be delivered intracorporally, as done in many electrophysiology studies. In the case of the intracorporal leads, these leads may be incorporated in a guidewire or catheter, such as those used to deliver the aspiration lumen and/or detector to the target site. By incorporating the EKG leads with a catheter and/or guidewire already used in the system, the system of the current invention becomes more seamless, with a lesser dependence on external components.

### Imaging-Based Inputs

Algorithms applied to image sequences, such as fluoroscopic image sequences, may be employed to identify the time of injection of a material that produces contrast in an image sequence. A representative embodiment of such an algorithm is one that detects a substantial change in the histogram of the density of pixels in each frame over time. The rate at which the contrast diffuses is subsequently calculated based on the rate of restoration of the histogram of pixel densities to its approximate baseline distribution (as per prior to injection of material). These two parameters are used to develop inputs into the controller of the aspiration mechanism. Other representative algorithms that may be applied include, but are not limited to: texture-based, histogram-based, derivative-based and motion-estimation algorithms. The employed algorithms may be dependent on the region of the anatomy that is imaged and may also accept EKG and/or respiratory signals as inputs to help the algorithm take into account any effects of motion of the region between image frames over the cardiac and/or respiratory cycles. Such algorithms may be deployed in real-time, or may be performed using post-processing on one of the first injections of the material that produces image contrast to help optimize the aspiration parameters for subsequent iterations of the removal of injected material. The advantage of the latter system is that it would have lower hardware requirements than a real-time system, but it would not produce information to the aspiration controller necessary for the iteration during which the optimized aspiration parameters were produced. The computational capabilities and interface circuitry necessary for the rapid optimization of aspiration parameters may not necessarily be incorporated directly into the aspiration controller itself, but may be incorporated in a software and/or hardware system that is either incorporated in the image acquisition device, or directly connected thereto. In this case, the system that detects the time of injection and/or rate of dispersion of the injected material may be configured to send input signals to the aspiration controller that describe its estimates or calculations of one or more of: the time of beginning of injection, the time of end of injection, the amount injected, time-course over which the injection occurred, the rate of dispersion of the material, the region to which the material flowed, the velocity of the leading edge of the material and other parameters. Alternatively, the input signals may directly tell the aspiration controller the time at which to begin aspiration, end aspiration and/or the degree to which aspiration should occur during that time period at either a uniform or variable rate. In this instance, a substantial part of the aspiration controller may be embedded in the system that does the image processing, and the cost of the aspiration controller, which may be a disposable component, can be minimized. The method of fluoroscopic detection of contrast has been reported in Mohammad-Reza Movahed. Removal of Iodine Contrast From Coronary Sinus in Swine During Coronary Angioplasty. J Am Coll Cardiol 2006;47:465-7; Sanaei-Ardekani M, Movahed M-R, Movafagh S, Ghahramani N: Contrast-Induced Nephropathy: A Review. Cardiovascular Revascularization Medicine. 2005;6:82-88; and Michishita et al., "A Novel Contrast Removal System from the Coronary Sinus Using an Adsorbing Column During Coronary Angiography in a Porcine Model," J.A.C.C. (2006) 47: 1866-1870. Aside from fluoroscopic-based image sequences, similar algorithms could be applied using ultrasound images, computed tomography, magnetic resonance images-and other modalities capable of rapidly sequential images over time (image acquisition rate rapid enough to observe the migration of the injected material), as long as the material injected contained a component that produces image contrast in the particular modality of imaging used.

### Fiber-Optic Based Inputs

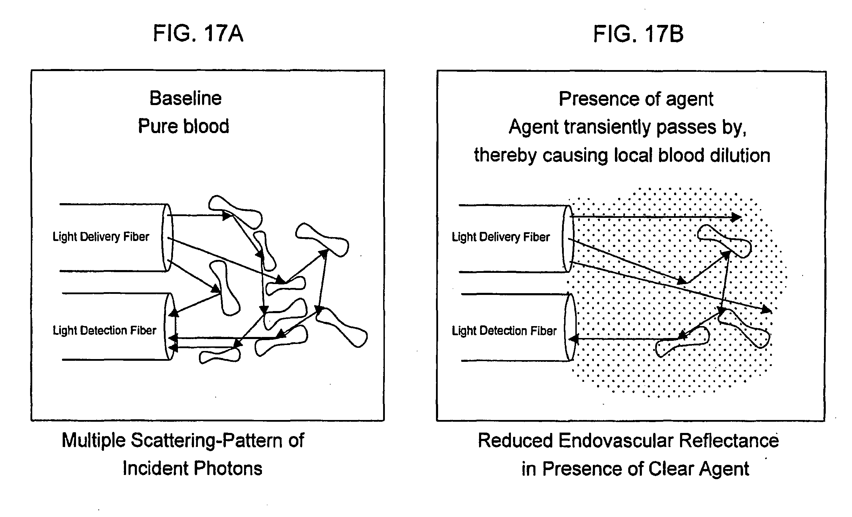

Certain embodiments of the system use one or more fiber-optic based sensors to detect the presence of the introduced material. Fiber optics are extremely cheap, versatile, disposable, biocompatible and non-conducting, making them an ideal material to use for an intracorporeal sensor. One or more fiber optics are delivered to the vicinity of the region from which material is to be aspirated, either via the one or more aspiration lumens, or alongside them. Alternatively, the fiber optics are incorporated into one or more of the aspiration lumens, or are delivered via lumen(s) included in the catheter(s) carrying the aspiration lumen(s). Regardless of the specific construction and mode of delivery of the fiber optic strand(s), a variety of modes of optically assaying the blood or other fluid in the vicinity of the region from which material to be aspirated can be used. Light of the ultraviolet, visible or infrared wavelengths can be transmitted down a fiber optic strand and used to illuminate a region near the distal end of the fiber. The interaction of light with the fluid, e.g., as shown in Figs. 17A and 17B, in that region can then be measured in several ways to provide information indicative of the composition of the fluid. Scattered or reflected light can be collected down either the same fiber or via another fiber. The scattered or reflected light may change in intensity or its composition in the electromagnetic spectrum or both and such changes can be detected by detectors at the proximal end of the fiber. An alternative embodiment would be to use one fiber to collect light that is emitted from another fiber and use the changes in the light's properties during transmission through the intervening fluid from an emitter to a detector to assess the fluid composition. In yet other embodiments, evanascent sensors are employed. Evanescent sensors may employ configurations as disclosed in U.S. Patent Nos.:5,750,337; 5,745,231; 5,633,724; 5,631,170; 5,192,510; 5,156,976;.4,893,894 and 4,852,967.

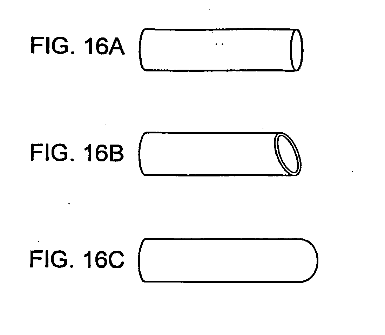

Where fiber-optic sensors are employed in which one or more fiber optic is optically coupled to a detector/illuminator positioned at a proximal location, the distal end of the fiber optic element may have a variety of configurations, e:g., as shown in Figures 16A to 16C. In Figure 16A, a plain tip is provided which may be employed in certain embodiments. In Figure 16B, a beveled tip is depicted, which may be employed in certain embodiments. Figure 16C provides a depiction of a non-traumatic tip (e.g. polished, rounded tip, which may be employed in certain embodiments of the invention. In certain embodiments, the tip configuration that is employed is configured to prevent clot formation, which may reduce performance of the optical system, where the tip configuration may be polished, rounded, coated, beveled tip, etc. In certain embodiments, the tip is configured to radiate (e.g., delivery fiberoptic) and/or collect (e.g., detection fiberoptic) a maximum amount of incident photons, where suitable configurations include polished, rounded, optimized index-matching, etc, e.g., as described in greater detail below.

Accordingly, a variety of different optic based detection systems or elements may be employed in the devices and protocols of the subject methods, where the optic based detection systems may evaluate transmitted and/or absorbed light in order to determine or evaluate a property, e.g., presence of target agent, in a fluid. As such, in certain embodiments one may perform a spectral analysis of light transmitted through and/or absorbed by a fluid. Alternatively, one may perform a spectral analysis of reflected/scattered light.

The properties of the interrogating light may be selected in order to provide for desired results. In certain embodiments, the spectral analysis may be made at one or more finite number of wavelength ranges, e.g., from about 200 nm to about 900 nm, from about 300 nm to about 900 nm, from about 450 nm to about 750 nm, from about 600 nm to about 700 nm, etc. In certain embodiments, the spectral analysis is made in range from about 430 nm to about 580 nm. In certain embodiments, the spectral analysis is made in range from about 595 nm to about 660 nm. In certain embodiments, the-spectral analysis is made in range from about 670 nm to about 780 nm. In certain embodiments, the spectral analysis is made in range from about 795 nm to about 900 nm. The detection system may include a single fiber optic embodiment or multi-fiber embodiment, where the system may include a reflective component. In certain embodiments, the light source employed is a laser. In certain embodiments, bright light may be employed.

Normal blood or other physiological fluid will have a measurable interaction with the emitted light that can either be known prior to the use of the device, or calibrated once the fiber optics are put in place, prior to the introduction of the material to be aspirated, so that a more anatomically specific and/or patient specific assessment of the baseline optical properties of that fluid is performed without the effects of the material to be introduced and aspirated. However, once the material to be introduced enters the region which is assayed by-the fiber optic system, the system will recognize a concentration-dependent change in the properties of the collected light. That information will be used as an input to the controller to trigger whether or not the aspiration is activated, and perhaps the extent of aspiration that is to occur. A major advantage of this system over a time-based system is that the ability to detect and use an elevation in the concentration of the material to be aspirated in order to trigger the aspiration provides a highly optimized system that removes only that physiological fluid, such as blood, which contains the highest concentrations of the material(s) to be removed.

In some cases, the material to be introduced may be of such a low-concentration, or have optical properties which are difficult to detect with sufficient sensitivity and/or specificity. Therefore, it may be desirable to include with the material to be introduced (first component) for therapeutic or diagnostic purposes one or more second components that would be introduced at the same time as the first material. Examples of such second components would include saline, which is clear in visible wavelengths,-or fluorescent compounds (or other labeled compounds, e.g., radiolabeled compounds, chromogenically labeled compounds, etc.) that produce specific wavelengths when photons of shorter wavelengths are presented to them, or pigmented compounds. In certain embodiments, the second component is capable of inducing a measurable physiological reaction in the target tissue, which reaction can be detected and thus initiate aspiration-mediated removal of fluid volume present at site of detection. The second component may be incorporated as a functional component of one or more of the molecules of the first component at a site that does not affect the active sites of the first component. The use of these second compositions is to serve as a tag to help identify that the batch of fluid which was introduced has migrated to the targeted region for aspiration by essentially improving the signal to noise ratio of the detection process. In those cases where it is known that there is a different mobility of the first and second components through the vascular beds or other anatomic structures, it may be necessary to assume a delay between the time at which the second component is detected and when the first component is assumed to have reached the target region, and that delay could be incorporated by the aspiration controller in determining the appropriate time to begin and end aspiration. In yet an alternative embodiment, the tag component and agent component may be present in a compartment or containment element, which compartment or containment element serves to keep the tag and agent components in a defined orientation or spatial relationship to each other. Compartment or containment elements of interest in which both the tag and agent components may be placed or packaged include, but are not limited to: microbubbles, liposomes, cells, etc.

Several fiber optic based detectors may be employed in certain embodiments to properly assay the target region's composition, e.g., in situations where it is possible that one or more of the sensors may appose an anatomical structure and therefore not be directed towards the fluid within the lumen or cavity of the structure. Such multi-sensor detectors may have a variety of different configurations. For example, the sensors may be placed at an offset upstream from the distal end of the aspiration ports in order to provide an earlier indication of when the material to be aspirated will arrive near the aspiration ports so that the system can more optimally time its activities via the aspiration controller. Similar sensors could be placed within the aspiration lumen(s) in order to assist in the quantification of the amount of fluid that was successfully retrieved by the system. In yet other embodiments, multiple detection fibers may be arranged around the circumference of a central emitting fibers, e.g., such that the multi-sensor optical detector has an annular configuration.

In certain embodiments where separate emitter and detector element are employed, the ends of the different elements may be coterminus or straggered. In certain embodiments, the fiberoptic sensor is fabricated from a material with a high numerical aperture, e.g., a numerical aperture ranging from about 0.1 to about 0.9, such as from about 0.2 to about 0.7.

In certain embodiments, a light collecting element, such as a deflective lens, may be positioned at a location, such as the distal end of the detector - element, which serves to guide reflected light from the fluid being assayed back to the detector.

In certain embodiments, the tip of fiberoptic detector is "polished" to produce a rounded shape of known dimensions and reflective/converging properties. These embodiments achieve two goals: Lense-effect for collecting higher number of reflectance photons, and Non-traumatic tip.

### Capacitance Based Detection

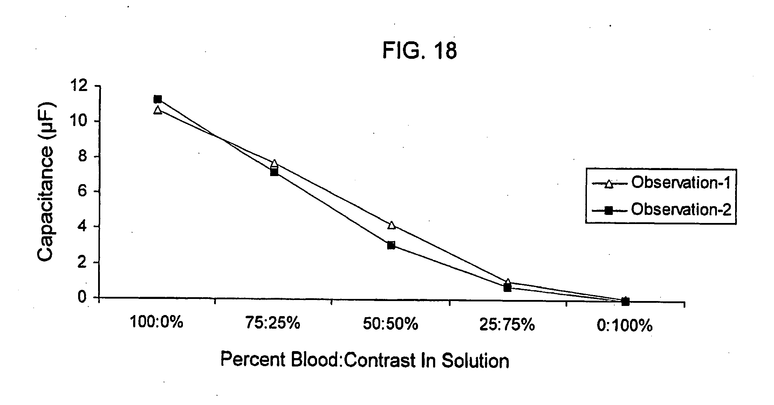

In certain embodiments, a capacitance based detection protocol is employed. For example, radiographic contrast is substantially less-ionized than blood, and this property can be assessed by capacitance measurement Therefore, capacitance of a given blood and contrast mixture can provide information about concentration levels of contrast in blood. Capacitance in mixtures of sheep's blood (packed cellular volume=37%) and radiographic contrast (lopamidol) of varying concentrations were measured. A total of 5 blood and contrast mixtures were prepared at the following concentrations: 100% blood, 75:25% blood/contrast, 50:50% blood/contrast, 25:75% blood/contrast, and 100% contrast. Capacitance measurements showed significantly different values between pure blood (>10µF) and pure contrast (<0.1µF). Additionally, there was an inverse relationship between contrast content in a blood solution and the corresponding capacitance measurement (See FIG. 18). Capacitance based measurement can therefore be utilized to provide information about contrast content in a blood solution after removal from the body. This system provides immediate quantitative measures on the amount of contrast removed from a patient. Real time feedback can be employed for assessing the procedure of removing contrast from the body

### Temperature Sensors

In yet other embodiments, temperature sensors, such as thermocouples and thermoresistors, are employed to detect the entrance of fluid with a slightly different temperature into the fluid collection site. In these embodiments, one or more temperature sensors are delivered to the target region of aspiration by the same methods as the fiber optics previously mentioned. The introduced fluid, or a substantial portion of it, has a temperature different from body temperature as it is introduced into the body. This temperature difference can be established by having the fluid at less than body temperature prior to being introduced, or the fluid could be heated slightly (e.g., to < 50°C) within or just prior to entering the injection catheter. As the introduced fluid travels through the capillaries or other small conduits between the site of introduction and the site of aspiration, there will be a substantial equalization of temperature of the fluid with the tissue through which it travels. However, the high precision of available temperature sensors is sufficient to detect the residual difference in temperature that-is expected as the introduced fluid enters the target aspiration region after its first pass through the perfused organ.

In certain embodiments, contrast agent that is allowed to rest for more than a few seconds within the lumen of the injection catheter within the body has sufficient time to equilibrate thermally with body temperature. This equilibration means that the initial volume of fluid to be introduced, approximately equal to the volume of the lumen of the catheter, would not be detectable as it enters the target aspiration region by thermal means. One method to overcome this limitation is to replace the column of fluid to be removed within the injection lumen(s) with a column of less harmful fluid, such as saline or blood, such that all the potentially harmful fluid to be introduced, such as contrast agent, would not be within the portion of the injection lumen(s) that is within the body prior to the initiation of injection. An alternative method is to heat fluid within the lumen near the very distal end of the injection lumen as it enters the body to establish a temperature gradient with an opposite polarity. This approach could be accomplished by incorporating an electric heating element in the distal end of the injection catheter.

### Acoustic Sensors

In yet other representative embodiments, one or more ultrasonic transducers are used in place of, or in addition to, either or temperature-based or fiber-optic based sensor. Such transducers may or may not have a mechanically rotating or translating motion capability, or have a phased-array functionality to control the direction of an emitted acoustic pulse. The transducers are employed to emit a series of acoustic pulses into the region near the distal region of the aspiration lumens. One or more of the transducers can be used to detect either backscattered or propagated acoustic energy. As blood or other physiological fluids in their pure forms are replaced with fluids that contain some of the material introduced upstream, there is a change in the intensity of the acoustic signals that are backscattered from and/or propagated through the region. Other changes of interest include changes in the slope of the frequency spectrum of the signal or changes in the statistical properties of the signal envelope. These changes are used to provide an indication of the presence of fluid laden with the material that was introduced upstream to trigger the aspiration mechanism via the controller.

### Binding Event Sensors

In certain embodiments, the sensor employed- is one that detects the presence of a particular agent of interest in the fluid by detection of a binding event, e.g., on a surface of the sensor. In such embodiments, a binding member immobilized on a surface of the sensor is employed to detect the presence of agent of interest in the fluid to be detected.

A variety of different binding agents may be employed for detection of agents (i.e., analytes) in a fluid. The binding member is an entity that specifically binds to an analyte of interest, e.g., the agent to be removed or a proxy agent therefore, such as described above. In certain embodiments, the binding member is an entity that has a high binding affinity for a second molecule. By high binding affinity is meant a binding affinity of about 10⁻⁴ M or higher, such as about 10⁻⁶ M or higher, e.g., 10⁻⁹M or higher. The binding member may be any of a variety of different types of molecules, so long as it exhibits the requisite binding affinity for the analyte or proxy therefore.

In certain embodiments, the binding member is a small molecule ligand. By small molecule ligand is meant a ligand ranging in molecular weight from about 50 to about 10,000 daltons, such as from about 50 to about 5,000 daltons and including from about 100 to about 1000 daltons. The small molecule may be any molecule, as well as a binding portion or fragment thereof, that is capable of binding with the requisite affinity to the analyte or proxy therefore. In certain embodiments, the small molecule is a small organic molecule that is capable of binding to the second molecule. The small molecule may include one or more functional groups necessary for structural interaction with the analyte or proxy therefore, e.g., groups necessary for hydrophobic, hydrophilic, electrostatic or even covalent interactions. The small molecule may also include a region that may participate in (or be modified to participate in) a covalent linkage to the surface of the sensor, without substantially adversely affecting the small molecule's ability to bind to the analyte or proxy therefore. Small molecule affinity ligands may include cyclical carbon or heterocyclic structures and/or aromatic or polyaromatic structures substituted with one or more of the above functional groups. Also of interest as small molecules are structures found among biomolecules, including peptides, saccharides, fatty acids, steroids; purines; pyrimidines, derivatives, structural analogs or combinations thereof. Such compounds may be screened to identify those of interest, where a variety of different screening protocols are known in the art. The small molecule may be derived from a naturally-occurring or synthetic compound that may be obtained from a wide variety of sources, including libraries of synthetic or natural compounds. For example, numerous means are available for random and directed synthesis of a wide variety of organic compounds and biomolecules, including the preparation of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known small molecules may be subjected to directed or random chemical modifications, such as acylation, alkylation, esterification, amidification, etc. to produce structural analogs. The small molecule may be obtained from a library of naturally occurring or synthetic molecules, including a library of compounds produced through combinatorial means, i.e., a compound diversity combinatorial library. When obtained from such libraries, the small molecule employed will have demonstrated some desirable affinity for the protein target in a convenient binding affinity assay. Combinatorial libraries, as well as methods for production and screening thereof, are known in the art and are described in United States Patent Nos.: 5,741,713; 5,734,018; 5,731,423; 5,721,099; 5,708,153; 5,698,673; 5,688,997; 5,688,696; 5,684,711; 5,641,862; 5,639,603; 5,593,853; 5,574,656; 5,571,698; 5,565,324; 5,549,974; 5,545,568; 5,541,061; 5,525,735; 5,463,564; 5,440,046; 5,438,119; 5,223,409.

In certain embodiments, the binding member may be a large molecule ligand. By large molecule is meant a ligand having a molecular weight greater than or equal to about 10,000 daltons. In certain embodiments, the large molecule ligand is an antibody, or binding fragment or mimetic thereof. Where antibodies are the large molecule ligand, they may be derived from polyclonal compositions, such that a heterogeneous-population of antibodies differing by specificity are employed, or monoclonal compositions, in which a homogeneous population of identical antibodies that have the same specificity for the target protein are employed. As such, the large molecule ligand may be either a monoclonal and polyclonal antibody. In yet other embodiments, the large molecule ligand is an antibody binding fragment or mimetic, where these fragments and mimetics have the requisite binding affinity for the target protein. For example, antibody fragments, such as Fv, F(ab)₂ and Fab may be prepared by cleavage of the intact protein, e.g., by protease or chemical cleavage. Also of interest are recombinantly-produced antibody fragments, such as single chain antibodies or scFvs, where such recombinantly produced antibody fragments retain the binding characteristics of the above antibodies. Such recombinantly-produced antibody fragments may include at least the VH and VL domains of the subject antibodies, so as to retain the binding characteristics of the subject antibodies. These recombinantly-produced antibody fragments or mimetics may be readily prepared using any convenient methodology, such as the methodology disclosed in U.S. Patent Nos. 5,851,829 and 5,965,371.The above-described antibodies, fragments and mimetics thereof may be obtained from commercial sources and/or prepared using any convenient technology.

In certain embodiments, the binding member is a nucleic acid. Nucleic acid domains for use in the subject methods are usually in the range of between about 20 up to about 1000 nucleotides in length, where in certain embodiments they may range from about 25 to about 500 nucleotides in length including from about 25 to about 250 nucleotides in length. The nucleic acid binding member moiety may be made up of ribonucleotides and deoxyribonucleotides as well as synthetic nucleotide residues that are capable of participating in Watson-Crick type or analogous base-pair interactions. The sequence of the nucleic acid affinity ligand is chosen or selected with respect to the sequence of the target molecule to which it binds.

Also suitable for use as binding member moieties are polynucleic acid aptimers. Polynucleic acid aptimers may be RNA oligonucleotides which may act to selectively bind proteins, much in the same manner as a receptor of antibody (Conrad et al., Methods Enzymol. (1996), 267(Combinatorial Chemistry), 336-367).

In these embodiments, in binding to the analyte or proxy therefore, the binding member may reversibly or irreversibly bind to the analyte or proxy therefore. In those embodiments where the binding member irreversibly binds to the analyte or proxy therefore, the detector may further be configured to provide for new, unbound binding member so as to be able to continuously detect the analyte or proxy therefore.

In these embodiments, the detecting of a binding interaction between the surface bound binding member and analyte or proxy therefore is employed to detect the presence of analyte. Any convenient protocol fro detecting the binding event may be employed. For example, evanescent based detection of the binding event may be employed, e.g., using configurations as described in Evanescent sensors may employ configurations as disclosed in U.S. Patent Nos.: 5,750,337; 5,745,231; 5,633,724; 5,631,170; 5,192,510; 5,156,976; 4,893,894 and 4,852,967.

### Other Sensors

Other sensors of interest include, but are not limited to: those that detect a change in pH of the fluid; a change in the dielectric constant between two electrically insulated leads where the fluid is found between the two electrical leads; a change in the conductivity of the fluid between two uninsulated leads through which a very low current is driven through their circuit which includes the fluid within its path; and changes in the magnetic properties of the fluid found between two coils or via a magnetic resonance imaging system; etc.

### Axially Positioned Sensors

In certain embodiments, a sensor is located on an element that extends along an internal distance of the aspiration lumen, e.g., on an element that is coaxially located on the central axis of the aspiration lumen, where the element may or may not extend beyond the distal end of the aspiration lumen. In such embodiments, configurations may be used which decrease resistance to fluid flow in the aspiration catheter, e.g., by reducing the profile of the sensor element located in the aspiration element. For example, the sensor positioning element may be present in the aspiration lumen in a "rapid-exchange" configuration, where the sensor position element exits the aspiration lumen at a port located at a region close to the distal end of the aspiration catheter, e.g., where the port may include a valve for providing a seal. Alternatively, a suitable cross-section, e.g., oval cross section, may be employed to reduce the profile of the sensor positioning element.

### Positioning and/or Retaining Mechanisms

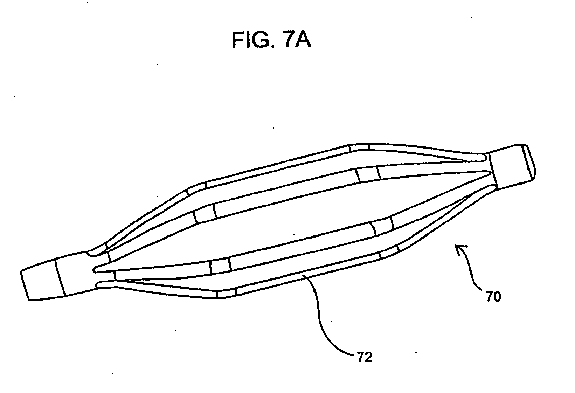

In some embodiments, a non-occlusive positioning and/or retaining mechanism is incorporated with either or both of the aspiration lumen and detector at the target region. For the purposes of this invention, a positioning mechanism is generally defined as a mechanism that tends to place elements of either an aspiration lumen or a detector in a more desirable general location than might otherwise occur. For example, it may center the aspiration lumen or detector within the target region. Such a mechanism might reduce the resistance to aspiration by distancing the one or more aspiration holes from the wall of the target region. It may improve the accuracy of detection by positioning a detector in a location that is more completely surrounded by the fluid in which the agent to be detected will likely be found. The detector may otherwise have difficulty in detecting the agent to be removed if the detector were in close proximity to the wall or other structures, of the targeted region. Furthermore, an expandable, non-occlusive mechanism, may serve the purpose of helping to retain the distal end of an aspiration lumen and/or detector within the targeted region of aspiration. Such a mechanism would assist in assuring the operator that the aspiration lumen will remain in the target region long enough to achieve the desired performance. A centering-anchoring mechanism according to an embodiment is shown in Fig. 7A.

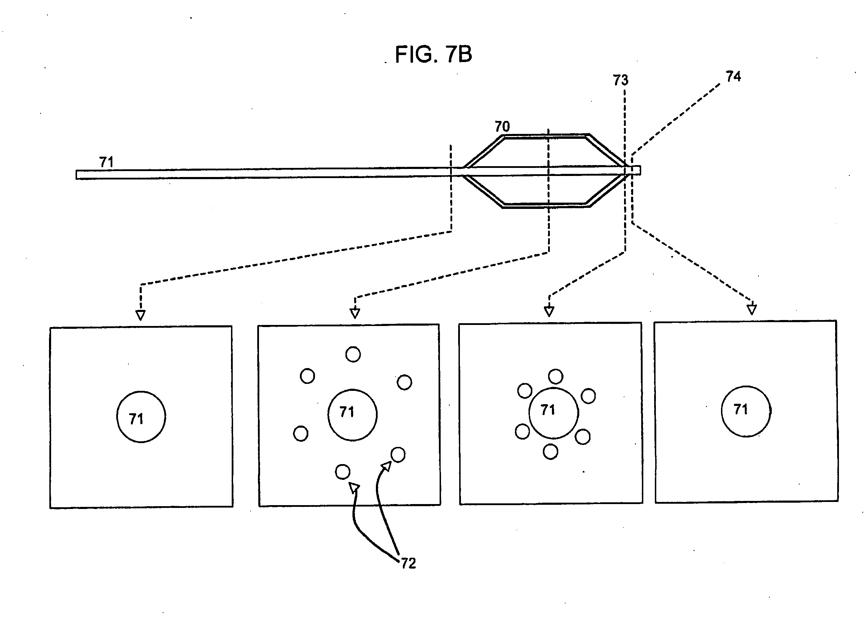

A more detailed view of an embodiment of a centering-anchor mechanism is provided in Fig. 7A. The centering-anchor mechanism depicted in Fig. 7A has a basket configuration that provides for an extended area of contact with a vessel wall when deployed. In Fig. 7A, centering-anchor device 70 includes tissue contact regions 72 which may vary in length, ranging in certain embodiments from about 2mm to about 40mm, such as from about 5mm to about 15mm, including from about 5mm to about 7mm. In certain embodiments, when the sensing element is pulled, the centering-anchor deploys in a manner that compresses regions 72 against the vessel wall. See also FIG. 7B

One application where a centering mechanism finds use is where the target region has a curved configuration, such as where the target location is present in a curved vessel, see e.g., Figs. 7F to 7G. In such embodiments, the centering mechanism may be employed to position the detector or a component thereof, e.g., the distal end of an optical fiber, a binding event sensor, etc., centrally within the lumen of the target site. Where the detector employs an optical fiber, this embodiment may direct the light beam coaxially into the lumen, e.g., to obtain more accurate results. In certain embodiments, the detector component is housed by the centering mechanism.

As reviewed above, the term detector is used broadly to refer to a variety of different types of detector devices. In certain embodiments, the detector may include a transducer located at the distal end, which detects the presence of the active agent at the distal end of the device and converts the detected presence to a signal, e.g., an electrical signal. In yet other embodiments, the detector at the distal end may be operably coupled to a remote transducer, e.g., a transducer located outside of the body. For example, in certain embodiments the detector is the end of an optical fiber; where the optical fiber transmits optical data from the detection point to a transducer, e.g., photodiode, that is located remotely from the efferent fluid collection site, e.g., at the proximal end of the device. For convenience in description, the term detector includes both embodiments where a transducer is or is not present at the efferent fluid collection site.

When employed, the centering device can have any convenient configuration that centers, and in some embodiments houses, the detector within the target site. Accordingly, the centering device can be of any shape and mechanism which achieves centering of the detector and directs a light beam toward the center of the vascular lumen. Representative configurations include, but are not limited to: flow modulating and non-flow modulating configurations, cylindrical or circular/oval configurations, straight or curved configurations, configurations of uniform or variable diameters, rigid or compliant configurations, metallic or non-metallic structures, self-expanding and expandable structures; anchoring configurations, such as stents, balloons, etc., non-anchoring configurations, including magnetic and stereotypically-guided configurations; permanent and temporary configurations, configurations where the centering mechanism is integrated with the detector (e.g., fiberoptic) device and configurations where the centering device is separate from the detector device; etc. The centering device may serve to position the detector component at a region which is completely surrounded by fluid in which a target agent is to be detected.

The detector can be positioned in a variety of locations relative to the centering device, as desired. In certain embodiments, the detector, e.g., end of a light guide such as an optical fiber, is positioned at the distal end of the centering mechanism. In yet other embodiments, the detector is positioned or housed inside of the centering device. In yet other embodiments, e.g., evananescent detection embodiments, the detector element is positioned at the proximal end of the centering mechanism.

Different embodiments of centering mechanisms are now described with reference to specific figures. Figure 7B shows a device having a catheter shaft 71 housing one or more optical fibers. The end of optical fiber 74 is positioned at the distal end of catheter shaft 71. Near the distal catheter tip is centering device 70, which device includes multiple distinct expandable members 72 that form an expandable basket. The distal tip of the centering device 73 is also shown.

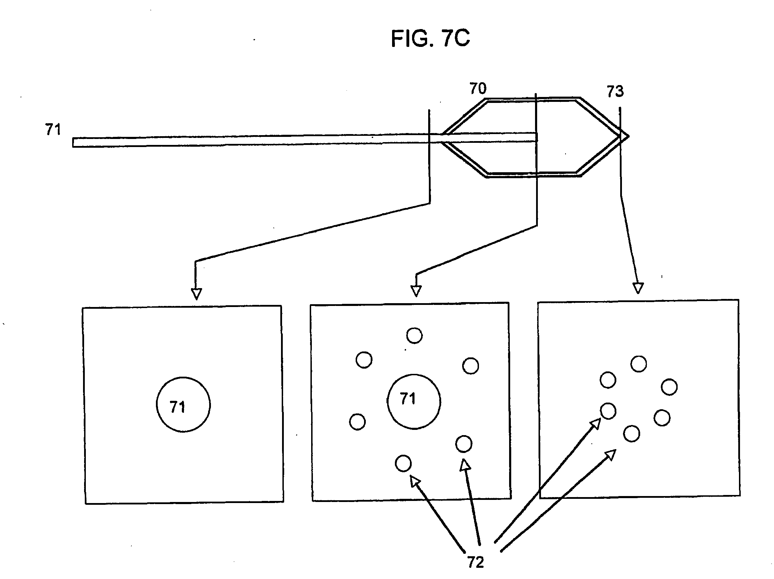

Fig. 7C provides a variation of the embodiment shown in Fig. 7B, where the end 74 of the optical fiber is present inside of the centering device. As shown in Fig. 7C, catheter shaft housing 71 is depicted which has one or more optical fibers. The end of the optical fiber 74 is present at the distal end of the catheter shaft. Also, shown is centering device 70, with expandable members 72. Cross-sections of centering device 70 are provided for the location where the detector is present and at the distal end, showing that the end 74 of the optical fiber is not present at the distal end 73 of the centering device. In certain of these embodiments, the centering device may include non-reflective surfaces, e.g., to reduce noise artifacts.

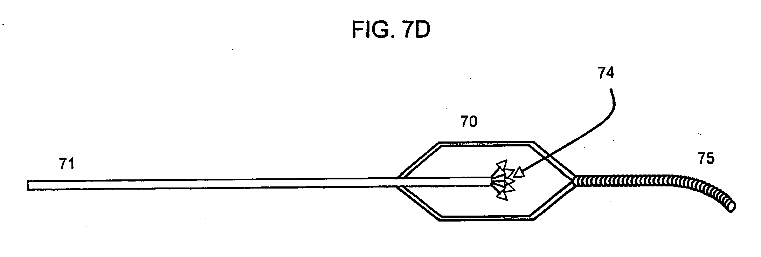

Fig. 7D provides a variation of the device shown in Fig. 7C, where the centering mechanism includes a distal floppy wire tip 75, e.g., to facilitate vascular advancement and navigation.

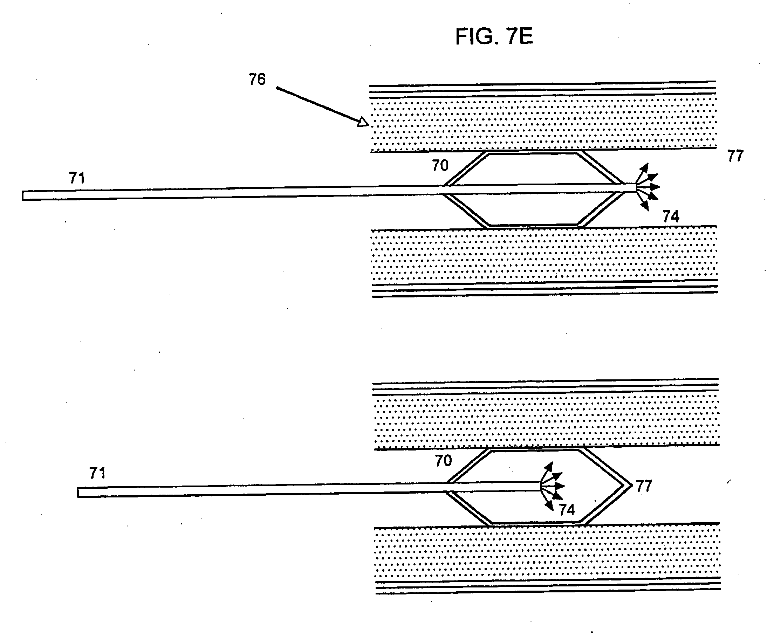

Fig. 7E shows the embodiments of Figs. 7B and 7C present inside non-curved vessels, respectively. As can be seen in Fig. 7E, centering mechanism 70 ensures that the optical fiber end 74 is positioned in the center of the lumen 77 of the vessel 76.

Fig. 7F, panel A provides a view of how the embodiment of Fig. 7C works in a target location present in a curved vessel. As shown in Fig. 7F, catheter shaft 71 houses the end 74 of an optical fiber. Also shown is the catheter shaft bias 71A produced in the curved vessel 76. Centering device 72 has distal tip 73, and maintains optical fiber end 74 in the center of vessel lumen 77. Also shown is intravascular valve 78. Fig. 7F, panel B shows the actual endovascular reflectance signal (= intensity of light backscatter). This signal was generated during in-vivo reflectance experiment in porcine coronary sinus. Trace 79A provides baseline endovascular reflectance signal which shows a significant signal drop 79B noticed as agent passes by illumination/detection point 74 inside centering device 70.

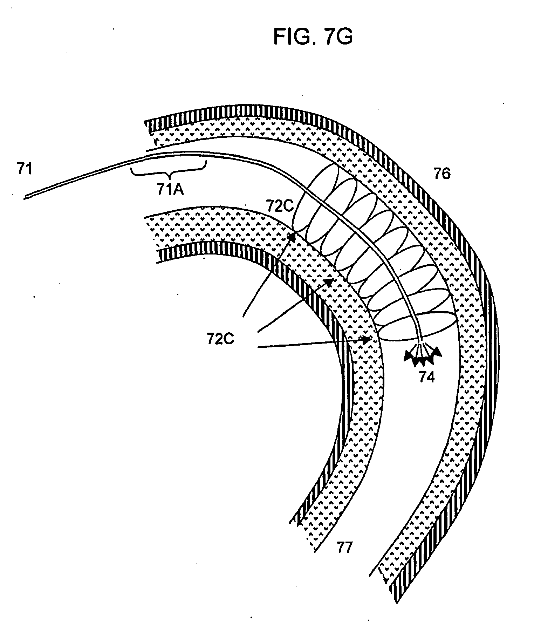

Fig. 7G, provides a view of an alternative embodiment of a centering device in a target location present in a curved vessel. As shown in Fig. 7G, catheter shaft 71 houses an optical fiber having distal end 74. Also shown is the catheter shaft bias 71A produced in the curved vessel 76. Centering device 72C is made up of multiple compliant expandable members, e.g., balloons, and maintains the optical fiber end 74 in the center of vessel lumen 77. The centering device 72C may be viewed as structure that adapts to vessel wall curvature, e.g., a segmented compliant balloon structure in the depicted embodiment.

### Signal Processing

The signal obtained from a given sensor may be used in raw form or processed in some manner. For example, in certain embodiments the signal is processed, e.g., to remove a noise component. In such embodiments, during reflectance sensing, noise signal related to fiberoptic cross-talk and pulsations of the vessel wall may create signal artifacts. Of interest here is signal noise created by the pulsation of the vessel wall. This noise can be reduced to minimum using methods such as: EKG-triggering, Farray phase transfer, back-end interferometry, or "chopping" of the signal at the level of lightsource.

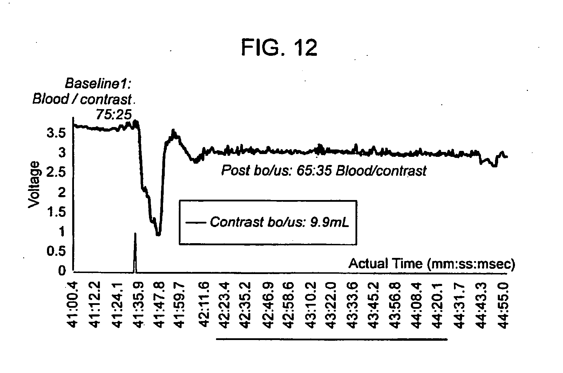

In certain embodiments, the system is configured to provide a visual display to a user of the agent detected by a sensor. For example, the system may be configured to provide a visual display, e.g., in the form of a graphical representation of agent concentration in a location of interest, e.g., the efferent fluid collection site, over time. For example, in reflectance based sensor systems, such a graph would show a drop in reflectance signal when the agent concentration increases locally, thereby causing a drop in reflectance signal. With proper calibration, the reflectance curve can provide direct information on the agent's concentration registered locally at the tip of the sensing element. This display would provide important information about the profile of agent entry into the efferent fluid collection site as represented by the shape of the curve, e.g., fast rise in concentration followed by slow wash-out etc. A display produced in certain embodiments is provided in Fig. 12. Any convenient graphical user interface technology may be employed.

In certain embodiments, concentration data output can be employed by the system to modulate aspiration parameters, e.g., to enhance efficiency of contrast removal. For example, the agent concentration curve can be used to control and modify aspiration efficiency, e.g., based on patient's condition, thereby weighing factors such as renal conditions vs. blood loss.

In certain embodiments where removed fluid is stored in an extracorporeal container, the concentration of agent in the extracorporeal container can be detected at one or more times, e.g., it can be monitored during the course of a given protocol. Any convenient manner of detecting agent concentration in the container may be employed. Ability to detect, such as quantify the amount of, agent (e.g., contrast) in an extra-corporeal container provides feedback to the user during a procedure that agent removal is successfully performed. Quantification of the removed volume can be weighed in during procedural decision-making.

In certain embodiments, signal processing algorithms may be employed for actuating and/or deactivating aspiration in an automated, e.g., closed-loop, fashion. For example, algorithms may be employed for slope detection, whereby changes in the magnitude, ratio, and/or frequency of sampling, may be employed to detect the presence or absence of agent in the efferent fluid collection site. Such signal processing algorithms find use in a number of embodiments. For example, an algorithm can be employed that turns on aspiration upon detection of agent and turns off aspiration at some predetermined time period following actuation. In a variation of this embodiment, the deactivations of aspiration may be provided by a processor having input from a different sensor, e.g., intra or extra-corporeal. In certain embodiments, the algorithm may be configured to make baseline adjustments, e.g., when evaluating raw data that includes a hysteresis component, e.g., as may be produced when the agent is a hyperemic agent. See e.g., Fig. 7F, panel B.

### Sealing Element

Regarding the flow pattern in the coronary sinus, it has been shown experimentally and clinically that a reflux of right atrial blood into the coronary sinus occurs. (D'Cruz IA, and Shirwany A. Update on echocardiography of coronary sinus anatomy and physiology. Echocardiography 2003;20:87-95). Therefore, in certain embodiments, the devices/systems may include a sealing element. For example, the device and systems made up of the devices may include an element that seals, either completely or partially, the efferent fluid collection site at a region downstream of the distal end of the aspiration lumen, e.g., so that fluid from a location downstream of the efferent fluid collection site is not drawn into the aspiration catheter during aspiration. In certain embodiments, the sealing element is configured to provide a seal (either a complete or partial seal) between the right atrium and coronary sinus at the site of the ostium so that blood does not flow into the coronary sinus from the right atrium during aspiration.

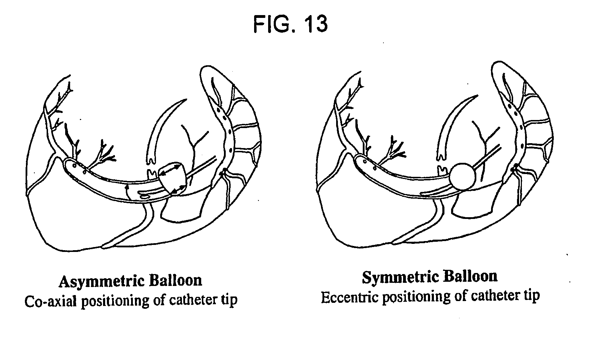

In certain embodiments, the sealing element is made up of an expandable element, such as a balloon, located at a position of the aspiration lumen that, upon inflation with the distal end of the aspiration lumen is located in the coronary sinus, seals the right atrium from the coronary sinus at the site of the ostium. In certain embodiments, the expandable element is an asymmetric right atrium balloon. Advantages of asymmetric include optimized positioning of catheter tip into coronary sinus, the coronary sinus connects to the right atrium tangentially with coronary sinus axis being wide-angulated (rather than perpendicular) to the wall of right atrium. An asymmetric balloon according to embodiments of the invention is depicted in Fig. 13. In these embodiments, the balloon has a length and/or diameter that provides for an asymmetric configuration upon inflation. In certain embodiments, the asymmetric balloon has a wedge-shape in longitudinal cross-section. In yet other embodiments, the aspiration catheter shaft runs eccentrically though the balloon, thereby producing radial asymmetry. In yet other embodiments, the balloon is a compliant, deformable balloon that provides for the desired flexibility for adapting to the right-atrial shape of the ostium of coronary sinus regardless of co-axiality. In certain of these embodiments, following aspiration upon detection of agent, the aspiration-induced, negative endovascular pressure will cause the balloon to move into sealing/abutting position onto ostium of target aspiration vessel (coronary sinus).

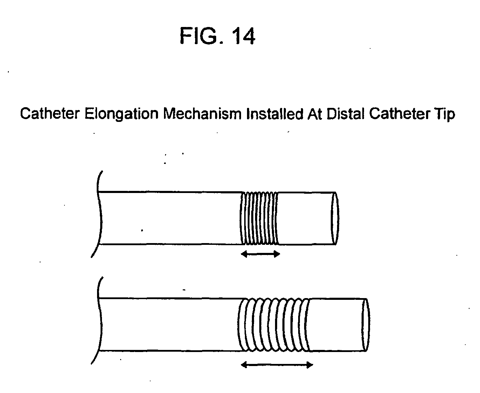

In certain embodiments, the aspiration lumen is present in a system that includes an element that forces a distal end balloon against the right atrium wall in a manner sufficient to seal the ostium. For example, a linear slide at the extra-corporeal segment of the aspiration lumen may be employed to compress a balloon against the wall of the right atrium and thereby produce a desired seal at the site of the ostium. Where desired, the "slide" mechanism can be manually operated and/or linked to a sensing element which actuates the mechanism automatically. In yet other embodiments, an elongation mechanism is provided at the distal end of the aspiration catheter. For example, a spring, telescope, or change in stiffness, e.g., which can be actuated in any convenient manner, e.g., manually, passively or automatically, may be employed to move the balloon to a sealing position at a desired time during aspiration. A representation of such an element is provided in Fig. 14.

### Flow Modulator Element

In certain embodiments, the devices include a flow modulator element for modulating fluid flow through the efferent fluid collection site, at least during removal of fluid therefrom. Accordingly, the device may include an element that changes or alters the nature of fluid flow through the collection site, e.g., by lengthening the fluid flow path, by narrowing the fluid flow path, by changing the velocity of fluid flow through the collection site, etc. For example, the device may include an expandable or deployable element, e.g., balloon, that can be deployed when positioned in the fluid collection site so as to alter a parameter of fluid flow through the site. Depending on the nature of the device and particular protocol being performed, the fluid flow modulation element may be positioned prior to, at substantially the same place as, or after the aspiration element.

### Fluid Exit Element

In certain embodiments, the subject devices include one or more fluid exit ports positioned downstream from the distal end of the aspiration element at which enters the aspiration lumen, but still at the distal end of the device. In certain embodiments, fluid flow through the fluid exit port or ports is controlled by a flow regulator element which can be moved at least between an "open" and a "closed" position, so that flow of fluid out of the aspiration element through the one or more exit ports can be controlled. A variety of different exit portion configurations may be present in these embodiments, including valves, closable windows, etc. Representative embodiments are further described below.

### Contrast Agent Removal System According to an Embodiment of the Invention

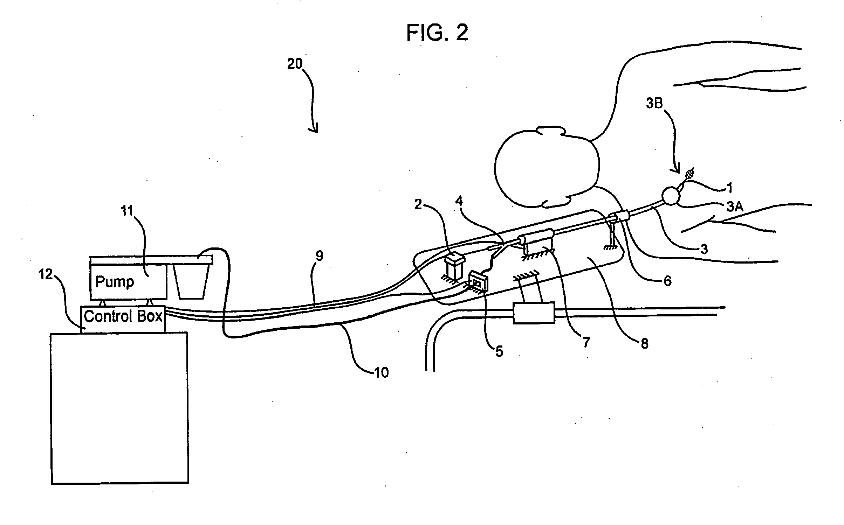

As reviewed above, aspects of the invention include devices and systems that include the same that are designed for removal of an agent from an internal efferent fluid collection site. Fig. 2 provides a depiction of a system according to an embodiment of the invention that is configured for the removal of contrast agent from the coronary sinus of a human, e.g., a human undergoing an cardiac imaging procedure. In Fig. 2, system 20 includes aspiration catheter 3 with a balloon element 3A located near distal end 3B. The distal end 3B of aspiration catheter 3 is located in the coronary sinus, while the balloon element 3A is present in the right atrium, e.g., in a manner that seals the ostium of the coronary sinus. Also, shown is sensing catheter 1 with centering-anchor element deployed. Aspiration catheter 3 is shown entering the human via introducer sheath 6. The outer diameter of the distal end of catheter 3 ranges in certain embodiments from about 1.0 mm to about 30 mm, such as from about 2 mm to about 7 mm. The inner diameter of the opening at the distal end of the aspiration catheter may range from about 1.0 mm to about 30 mm, such as from about 2 mm to about 7 mm.

Also shown in system 20 is datum plate 8 which provides a number of the system components. Present on datum plate 8 is actuator 7, which actuator provides for movement of the aspiration catheter in the human. Connected to actuator 7 is hemostasis valve 4. Shown exiting hemostasis valve 4 is the sensing catheter which terminates at fiber optic base 2. Also shown exiting hemostasis valve 4 is vacuum line 10 which terminates in a reservoir of pump 11. Control box 12 provides for signal communication with fiber optic base 2 via cable bundle 9 and with aspiration valve 5 via line 13.

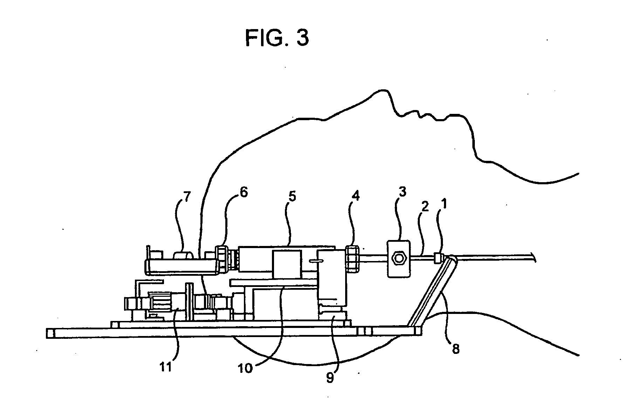

Fig. 3 provides a detailed view of certain components of the datum plate of system 20. Shown in Fig. 3 is introducer sheath 1 held in place by fixation arm 8. Entering introducer sheath 1 is aspiration catheter 2. Also shown is catheter torque handle 3 with valve, hemostatic valve for aspiration catheter 4, catheter handle 5, hemostatic valve for sensor wire 6 and light source 7. Element 9 is a pinch valve for regulating the aspiration vacuum and therefore aspiration through the aspiration catheter 2. Element 10 is a linear slide plate for moving the aspiration catheter 2 against the introducer sheath 1 resulting in forward movement of the aspiration catheter 2 inside the heart (e.g., to provide a seal between the right atrium and the coronary sinus at the ostium. Motor 11 provides for movement of the linear slide 10 in the desired direction.

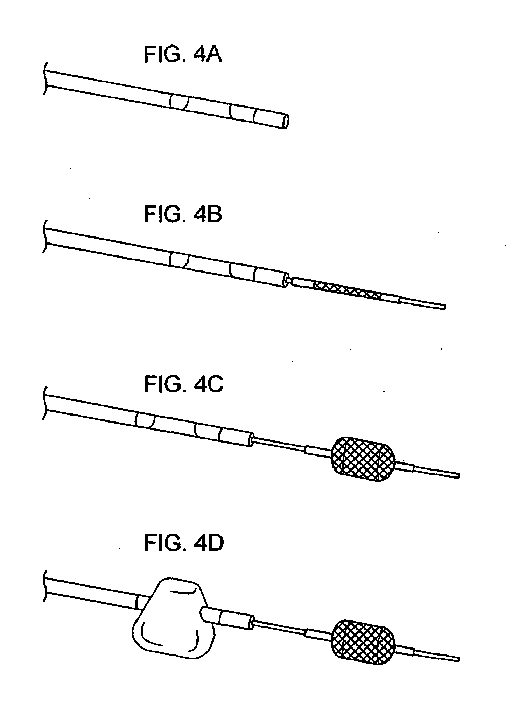

Figs. 4A to 4D provide a sequential view of the distal end of the aspiration catheter as it is being deployed following introduction into the coronary sinus. In Fig. 4A, distal end of aspiration catheter is shown as it would be extending from the right atrium into the coronary sinus. In Fig. 4B, sensor-catheter which is coaxial to the aspiration catheter is shown extended beyond the end of the aspiration catheter. In Fig. 4C, a centering-anchor mechanism on the sensor catheter is shown in deployed format, which holds the sensor catheter in place in the coronary sinus. In Fig. 4D, asymmetrical balloon on the aspiration catheter is deployed, providing for a seal between the right atrium and the coronary sinus at the site of the ostium.

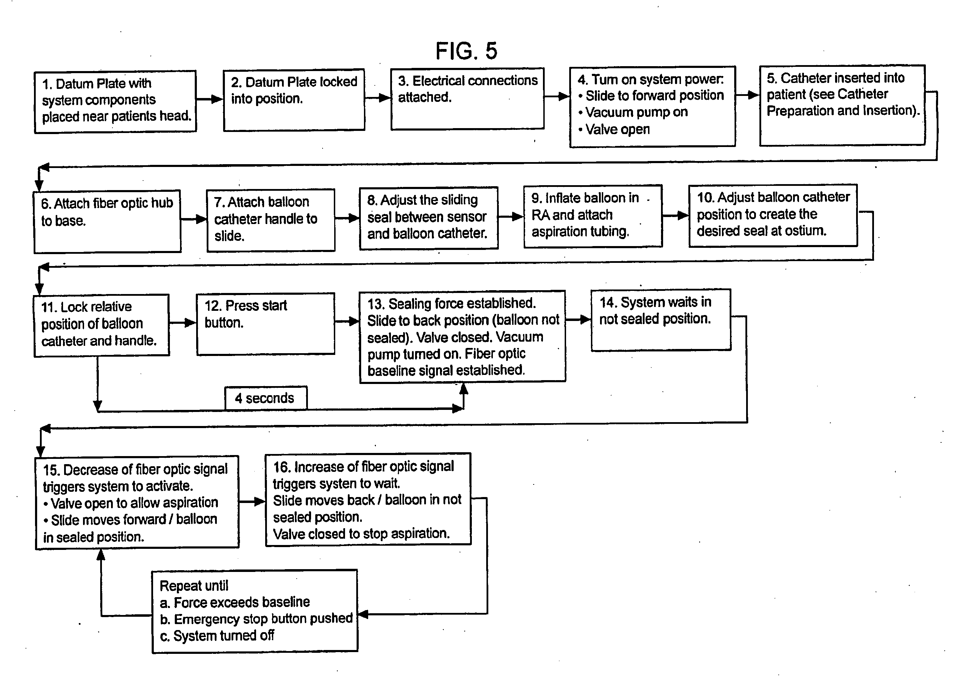

Fig. 5 provides a flow diagram of how the system depicted in Figs. 2 to 4 may be operated to selectively remove contrast agent from the coronary sinus of a human.

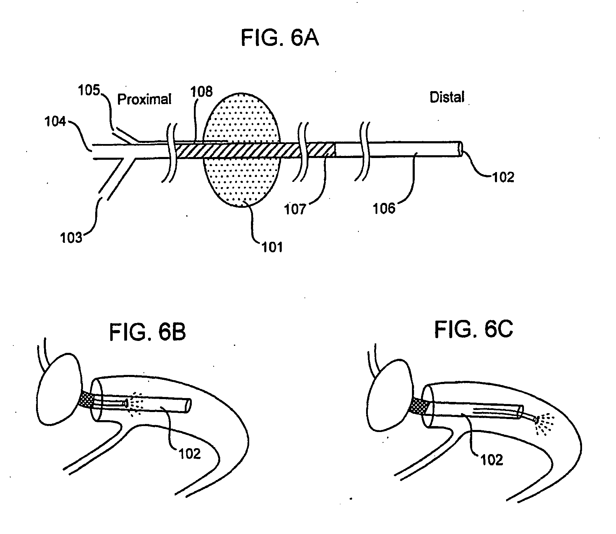

Fig. 6A provides another view of the aspiration catheter shown-in the embodiments of Figs. 2 to 4. In Fig. 6A, aspiration catheter 2 includes proximal end having aspiration port 103, balloon inflation port 105 and sensor wire port 104. Also shown is balloon inflation channel 108 in communication with right atrial balloon element 101. Region 107 is a non-transparent braided catheter shaft, while region 106 (positioned in the coronary sinus) ending at distal end 102 is a light transparent segment. Since region 106 is transparent, the detector end of a coaxial sensor catheter may be extended beyond the distal end of-the aspiration catheter, e.g., as shown in Fig. 6C or be positioned within the transparent region as shown in Fig. 6B and still detect agent when it enters the coronary sinus.

As shown in Figs. 4A to 4D, the sensor catheter includes a centering-anchor mechanism for stably holding the sensor catheter in place when present in the coronary sinus. A more detailed view of an embodiment of a centering-anchor mechanism is provided in Fig. 7A. The centering-anchor mechanism depicted in Fig. 7A has a basket configuration that provides for an extended area of contact with a vessel wall when deployed. In Fig. 7A, centering-anchor device 70 includes tissue contact regions 72 which may vary in length, ranging in certain embodiments from about 2mm to about 40mm, such as from about 5mm to about 15mm, including from about 5mm to about 7mm. When the sensing element t is pulled, the centering-anchor deploys in a manner that compresses regions 72 against the vessel wall.

As mentioned above, the sensing element can have a number of different configurations. For example, in optical sensing elements, the sensing element may be a reflectance sensor, a transmission sensor, an evanescent sensor, etc., as reviewed above.

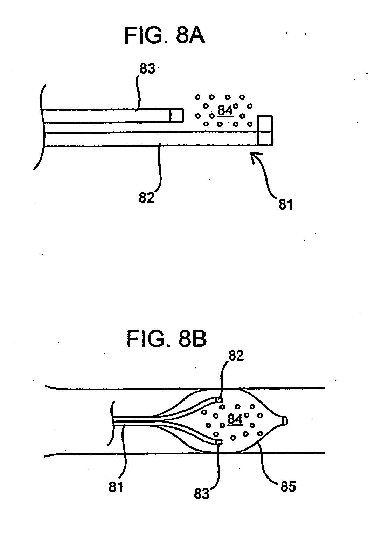

In certain embodiments, the sensor is a transmission sensor, in that light that is emitted and passes through a fluid medium is then detected by a detector in order to provide a signal that is used to determine a parameter of the fluid, e.g., the presence of contrast agent in the fluid. Such a transmission sensor can assume a number of different configurations. Fig. 8A provides view of an embodiment where the detector includes separate sensor and emitter fibers (e.g., an example of a dual fiber detector system) which are not coterminus, where fluid (e.g., blood containing red blood cells (RBC) can flow between the emitter 82 and sensor 83 fibers. In the embodiment shown in Fig. 8A, the emitter fiber extends beyond the sensor fiber. However, the reverse configuration in which the sensor fiber extends beyond the emitter fiber may also be employed. Blood (denoted by redblood cells 84) is present between the emitter 82 and the sensor 83.

Fig. 8B provides another view of another embodiment of a transmission based sensor 81, which is a dual fiber sensor. In Fig. 8B, the emitter 82 and detector 83 fibers of the sensor are present on distinct fibers that extend the same length between the distal end of the aspiration catheter. When deployed, the emitter and detector elements are positioned adjacent the vessel walls, e.g., by a flow through design anchoring structure 85, such that blood flows between the two elements. In the embodiments shown in Figs. 8A and 8B, the detector and emitter elements/fibers may be associated with the tissue contact regions of a flow through centering-anchor structure, e.g., as shown in Fig. 7A, denoted in FIG. 8B as element 85. As such, upon deployment of the centering-anchor, the detector and emitter elements will be positioned at appropriate locations to make transmission measurements of blood flowing through the centering-anchor device.

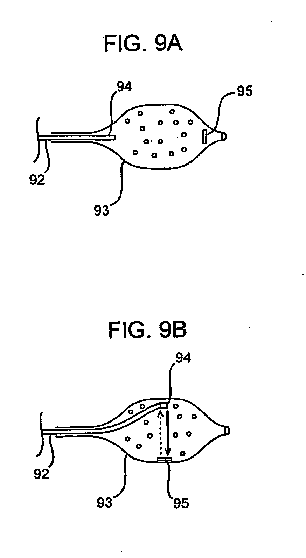

In certain embodiments, instead of a dual fiber transmission sensor, a single fiber transmission sensor that employs a reflective element may be employed. Embodiments of single fiber transmission sensors are shown in Figs. 9A and 9B. In the embodiment shown in Figs. 9A and 9B, the detector 91 includes a single emitter/detector fiber 92 with a sensor 94 at its distal end. A centering mechanism 93 is also present. Positioned either axially (see Fig. 9A) or radially (see Fig. 9B) opposite the sensor is a reflecting element (e.g., mirror) 95, positioned relative to the end of the emitter/detector fiber such that light leaving the end of the fiber is reflected by the reflective element back to the fiber, and then detected.

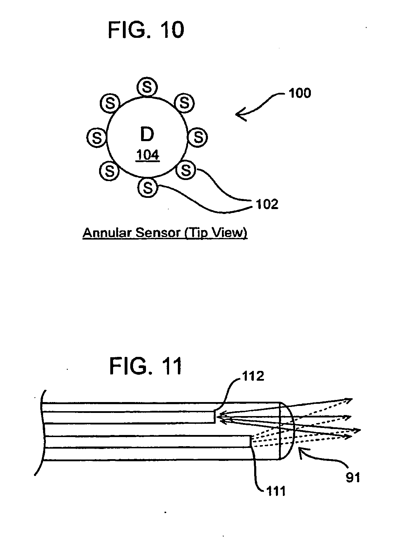

As described above, in certain embodiments the sensor may actually be a multi-detector structure. Fig. 10 provides a cross-sectional view of the distal end 100 of a multi-detector structure according-to an embodiment of the invention. In the sensor structure shown in Fig. 10, the sensor includes multiple detectors (s) 102 arranged circumferentially around central emitter (D) 104. Advantages of multiple, annular detector fibers arranged around delivery fiberoptic as shown in Fig. 10 include maximization of capturing of reflectance signal. In a reflectance-based catheter system, approximately 70% of the back-scattered photons are crossing within 1.25mm range from the center of the delivery fiber. This indicates that annular detector is best under these conditions.

As reviewed above, in certain embodiments the sensor elements may be present in a structure that includes a deflective lens at its tip. An embodiment of such a structure is shown in Fig. 11. In Fig. 11, deflective lens 91 serves two purposes. First, deflective lens 91 expands the range of emitted light from the emitter 111 and also collects and guides the light to the detector 112. Second, the lens reduces the thrombogenicity of the end of the sensor by providing a smoothed end.

### SYSTEMS

Also provided are systems for use in practicing the subject methods, where the systems include a device for selectively removing agent from the efferent fluid collection site, such as the representative devices described above, and may optionally include one or more additional components that find use in practicing the subject methods, e.g., detectors, agent introducers, data recorders, etc. A representative system is depicted in Figure 1. In the system depicted in Figure 1, the system includes the standard device components, i.e., an aspiration controller 11 and aspiration mechanism 12 operatively linked to an aspiration lumen which is introduced into the subject (body) 13, as well as a number of additional/optional components, such as an injection/delivery system **14** for introducing agent into the body at a site upstream of the target efferent fluid collection site, one or more detector elements 15 for detecting the presence of agent in the efferent fluid collection site, and an aspiration recorder/display element 16 for recording data (e.g., fluid flow data, etc.) and displaying the same to the operator. A system according to another embodiment of the invention is shown in Fig. 2.

### UTILITY

Embodiments of the invention find use in a wide variety of different applications, including both diagnostic and therapeutic applications. Of particular interest is the use of embodiments of the methods and devices to selectively remove from a patient a locally administered diagnostic or therapeutic agent, so that the patient is not systemically exposed to the diagnostic or therapeutic agent. In certain embodiments, embodiments of the methods are employed to selectively remove a locally administered diagnostic agent, such that the diagnostic agent is only contacted with a limited region or portion of the patient to which it is administered, e.g., a specific organ or portion thereof. A common example of such a compound is radio-opaque dye. Iodinated forms of such a dye are used routinely during catheter-based interventional procedures such as coronary, renal, neurological and peripheral arteriography. The iodine component has a high absorption of x-rays and therefore provides a contrast medium for the radiological identification of vessels when introduced within an upstream artery. However, the use of such dyes is known to have potential toxic effects depending on the specific formulation, including direct injury to renal tubule cells, endothelial injury, bronchospasm, inflammatory reactions; pro-coagulation, anti-coagulation, vasodilation and thyrotoxicosis.

Another utility of embodiments of the invention is the selective removal from a patient of a locally administered therapeutic agent, where representative therapeutic agents or materials that may be introduced locally for desired effects but whose direct or other effects would be undesired elsewhere include vasoactive agents, cytotoxic agents, genetic vectors, apoptotic agents, anoxic agents (including saline), photodynamic agents, emboli-promoting particles or coils, antibodies, cytokines, immunologically targeted agents and hormones. Additional agents of interest include, but are not limited to: cells, enzymes, activators, inhibitors and their precursors, as well as sclerosing agents, antiinflammatories, pro-inflammatories, steroids and osmotic agents; and the like. As such, another application of the subject methods is to determine the amount of agent retained at a local area or region of a subject upon local administration of the agent to the subject. For example, where a therapeutic agent is locally administered to a region or location of a subject, e.g., an organ, and blood carrying the agent is selectively removed from the subject according to the subject methods, the amount of agent in the collected blood can be used to determine the amount of agent that was retained by the local region or area, e.g., organ, of the subject. As such, in those cases where the present invention is used to retrieve a diagnostic or therapeutic agent for which a portion of that agent desirably resides in the region into which it is delivered, and the portion of the agent collected from the collection represents an amount of the agent that did not remain resident in that region, the subject methods may be employed to estimate the effective dosage of the agent. For example, in the localized delivery of a chemotherapeutic agent via the afferent branches of a targeted tumor, the present invention is capable of collecting some of the chemotherapeutic agent before it is able to enter into the systemic circulation, thus minimizing its side effects. The difference between the amount of agent injected and the amount of agent that is retrieved by the present invention represents the sum of the amount of agent that was successfully incorporated into the tumor and the amount of agent that escaped to the systemic circulation. If a goal of the localized delivery of the chemotherapeutic agent is to attempt to incorporate a given dosage of the agent into the tumor, it is possible to use the present invention to better estimate how much of the delivered agent was successfully incorporated into the tumor by estimating how much of the agent was retrieved in the collection site. If a higher than expected amount of agent was retrieved in the collection site, than a substantial portion of the agent was not successfully incorporated into the tumor and this may direct the physician to deliver more agent to the tumor, or consider alternative strategies for treatment. The higher the efficacy of the present invention is in terms of retrieving the agent, the more accurate the estimate of the amount of agent successfully delivered to the site will become.

### KITS

Also provided are kits for use in practicing embodiments of the methods, where the kits may include one or more of the above devices, and/or components of the subject systems, as described above. As such, a kit may include a device, such as a catheter device, that includes an aspiration lumen, aspiration mechanism and aspiration mechanism controller, as described above. The kit may further include other components, e.g:, guidewires, etc, which may find use in practicing the subject methods.

In addition to the above-mentioned components, the subject kits may further include instructions for using the components of the kit to practice the subject methods. The instructions for practicing the subject methods may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

Accordingly, the preceding merely illustrates the principles of the invention. It will be appreciated that those skilled in the art will be able to devise various arrangements which, although not explicitly described or shown herein, embody the principles of the invention and are included within its scope. Furthermore, all examples and conditional language recited herein-are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of present invention is embodied by the appended claims.

## Claims

1. A system for selectively removing an agent from a physiological efferent fluid collection site, said site being a vascular fluid collection site, said system comprising:
(a) a non-occlusive aspiration lumen being configured such that a distal end can be positioned in the vascular fluid collection site;
(b) an aspiration mechanism operatively connected to said non-occlusive aspiration lumen;
(c) an actuation controller element for controlling actuation of said aspiration mechanism; and
(d) a sensor for sensing agent at a target sensing site, **characterised in that** the sensor is located distal to a distal opening of the aspiration lumen.

2. The system according to Claim 1, wherein the actuation controller element is arranged for controlling actuation of said aspiration mechanism in response to detection of the agent at the target sensing site.

3. The system according to claim 1 or 2, wherein said sensor is an optical sensor.

4. The system according to Claim 3, wherein said sensor is one of the following:
• a dual fiber sensor,
• is a single fiber sensor,
• a transmission sensor,
• a reflectance sensor,
• an evanescent sensor,

5. The system according to any of the preceding claims, further comprising a centering mechanism, preferably wherein the sensor is present on the centering mechanism.

6. The system according to any of the preceding claims wherein said aspiration element further comprises an elongation mechanism at its distal end.

7. The system according to any of the preceding claims wherein said aspiration element further comprises a transparent distal region.

8. The system according to any of the preceding claims further comprising a shunting element.

9. The system according to any of the preceding claims further comprising a pressure sensor.

10. The system according to any of the preceding claims further comprising an extracorporeal fluid treatment element, preferably wherein said extracorporeal fluid treatment element comprises a filter.

11. The system according to any of the preceding claims further comprising one or more of the hollowing:
• supporting means for supporting one more tributaries at the efferent fluid collection site,
• monitoring means, for monitoring fluid flow through the efferent fluid collection site,
• signal processing means for processing signals from the sensor,
• sealing means, for example for the effluent collection site at a region downstream the distal end of the aspiration lumen,
• a flow modulator for modulating fluid from through an effluent collection site,
• a fluid exit element,
• means for extracorporally treating a removed agent, for example wherein the means comprise a filter.

12. The system according to any of the preceding claims, further comprising an agent, for example a diagnostic agent or contrast agent, removed from a patient.

13. System according to any of the preceding claims for selectively removing an agent from a physiological efferent fluid collection site, and optionally, subsequently extracorporally treating said removed agent.

14. A kit of parts comprising a system according to any of the preceding claims and instructions for selectively removing an agent from a physiological efferent fluid collection site, and optionally, subsequently extracorporally treating said removed agent.

15. A method for extracorporally treating an agent removed from a physiological effluent collection site, said site being a vascular fluid collection site, said method comprising using a system or kit according to any of the preceding claims.

## Patentansprüche

1. System zum selektiven Entfernen eines Agents von einer physiologischen, efferenten Flüssigkeitssammelstelle, wobei die Stelle eine Gefäßflüssigkeitssammelstelle ist und das System Folgendes aufweist:
(a) ein nicht-okklusives Aspirationslumen, welches so gestaltet ist, dass ein distales Ende an der Gefäßflüssigkeitssammelstelle positioniert sein kann;
(b) einem Aspirationsmechanismus, welcher mit dem nicht-okklusiven Aspirationslumen verbunden ist;
(c) einem Betätigungssteuerelement zum Steuern der Betätigung des Aspirationsmechanismus; und
(d) einem Sensor zum Detektieren des Agens an einem Zielmessort, **dadurch gekennzeichnet**, pass der Sensor distal zu einer distalen Öffnung des Aspirationslumen angeordnet ist.

2. System nach Anspruch 1, wobei das Betätigungssteuerelement zum Steuern der Betätigung des Aspirationsmechanismus nach Detektion des Agens am Zielmessort ungeordnet ist.

3. System nach Anspruch 1 order 2, wobei der Sensor ein optischer Sensor ist.

4. System nach Anspruch 3, wobei der Sensor einer der folgenden ist:
- ein Doppelfasersensor
- ein Einzelfasersensor
- ein Transmissionssensor
- ein Reflexionssensor
- ein Evaneszenzsensor

5. System nach einem der vorhergehenden Ansprüche, des Weiteren aufweisend einen Zentriermechanismus, wobei der Sensor vorzugsweise auf dem Zentriermechanismus angesiedelt ist.

6. System nach einem der vorhergehenden Ansprüche, wobei das Aspirationselement des Weiteren am distalen Ende einen Verlängerungsmechanismus enthält .

7. System nach einen der vorhergehenden Ansprüche, wobei das Aspirationselement des Weiteren einen transparenten, distalen Abschnitt enthält.

8. System nach einem der vorhergehenden Ansprüche, welches des Weiteren ein Shunt-Element enthält.

9. System nach einem der vorhergehenden Ansprüche, welches des Weiteren einen Druckfühler enthält.

10. System nach einem der vorhergehenden Ansprüche, welches des Weiteren ein extrakorporales Flüssigkeitsbehandlungselement enthält, wobei das Flüssigkeitsbehandlungselement vorzugsweise einen Filter enthält.

11. System nach einem der vorhergehenden Ansprüche, des Weiteren mindestens eine der folgenden Einrichtungen aufweisend:
- eine unterstützende Einrichtung zur Unterstützung eines weiteren Nebenflusses an der efferenten Flüssigkeitssammelstelle,
- eine Überwachungseinrichtung zur überwachung des Flüssigkeitsflusses durch die efferente Flüssigkeitssammelstelle,
- eine signalverarbeitende Einrichtung zur Signalverarbeitung durch den Sensor,
- eine verschließende Einrichtung, zum Beispiel für die abfließende Flüssigkeitssammelstelle in einem Bereich abwärts des distalen Endes des Aspirationslumen,
- einen Flussmodulator zur Modulation der Flüssigkeit durch die abfließende Flüssigkeitssammelstelle,
- ein Element für den Flüssigkeitsaustritt,
- eine Einrichtung zur extrakorporalen Behandlung eines entfernten Agens, wobei diese Komponente zum Beispiel einen Filter enthält.

12. System nach einem der vorhergehenden Ansprüche, des Weiteren aufweisend ein Agens, zum Beispiel ein Diagnostikum oder ein Kontrastmittel, welches aus dem Patienten entfernt wird.

13. System nach einem der vorhergehenden Ansprüche zum selektiven Entfernen eines Agens von einer physiologischen, efferenten Flüssigkeitssammelstelle und optional zum anschließenden extrakorporalen Behandeln des entfernten Agens.

14. Ein Kit bestehend aus einem System nach einem der vorhergehenden Ansprüche und Angaben zum selektiven Entfernung eines Agens von einer physiologischen, efferenten Flüssigkeitssammelstelle und optional zum anschließenden extrakorporalen Behandeln des entfernten Agens.

15. Ein Verfahren zum extrakorporalen Behandeln eines Agens, welches von einer physiologischen, efferenten Flüssigkeitssammelstelle entfernt wurde, wobei die Stelle eine Gefäßflüssigkeitssammelstelle ist und besagtes Verfahren den Gebrauch eines Systems oder Kits nach einem der vorhergehenden Ansprüche einschließt.

## Revendications

1. Système pour extraire sélectivement un agent d'un site de collecte de fluides efférents physiologiques, ledit site étant un site de collecte de fluides vasculaires, ledit système comprenant :
(a) une lumière d'aspiration non occlusive configurée de sorte qu'une extrémité distale peut être positionnée dans un site de collecte de fluides vasculaires ;
(b) un mécanisme d'aspiration raccordé de manière opérationnelle à ladite lumière d'aspiration non occlusive ;
(c) un élément d'organe de commande d'actionnement pour commandeur l'actionnement dudit mécanisme d'aspiration ; et
(d) un capteur pour détecter l'agent sur un site de détection cible, **caractérisé en ce que** le capteur est positionné à distance d'une ouverture distale de la lumière d'aspiration.

2. Système selon la revendication 1, dans lequel l'élément d'organe de commande d'actionnement est agencé pour commander l'actionnement dudit mécanisme d'aspiration en réponse à la détention de l'agent sur le site de détection cible.

3. Système selon la revendication 1 ou 2, dans lequel ledit capteur est un capteur optique.

4. Système selon la revendication 3, dans lequel ledit capteur est l'un des suivantes :
un capteur à deux fibres,
un capteur à fibre unique,
un capteur de transmission,
un capteur de réflectance,
un capteur évanescent.

5. Système selon l'une quelconque des revendications précédentes, comprenant en outre un mécanisme de centrage, de préférence dans lequel le capteur est présent sur le mécanisme de centrage.

6. Système selon l'une quelconque des revendications précédentes, dans lequel ledit élément d'aspiration comprend en outre un mécanisme d'allongement au niveau de son extrémité distale.

7. Système selon l'une quelconque des revendications précédentes, dans lequel ledit élément d'aspiration comprend en outre une région distale transparente.

8. Système selon l'une quelconque des revendications précédentes, comprenant en outre un élément de dérivation.

9. Système selon l'une quelconque des revendications précédentes, comprenant en outre un capteur de pression.

10. Système selon l'une quelconque des revendications précédentes, comprenant en outre un élément de traitement de fluide extracorporel, de préférence dans lequel ledit élément de traitement de fluide extracorporel comprend un filtre.

11. Système selon l'une quelconque des revendications précédentes, comprenant en outre un ou plusieurs des éléments suivants :
des moyens de support pour supporter un affluent de plus sur le site de collecte de fluides efférents,
des moyens de surveillance pour surveiller l'écoulement de fluide à travers le site de collecte de fluides efférents,
des moyens de traitement de signal pour traiter des signaux provenant du capteur,
des moyens d'étanchéité, par exemple pour le site de collecte d'effluents dans une région en aval de l'extrémité distale de la lumière d'aspiration,
un modulateur d'écoulement pour moduler le fluide dans le site de collecte d'effluents,
un élément de sortie de fluide,
des moyens pour traiter de manière extracorporelle un agent extrait, par exemple dans lequel les moyens comprennent un filtre.

12. Système selon l'une quelconque des revendications précédentes, comprenant en outre un agent, par exemple un agent de diagnostic ou un agent de contraste, extrait d'un patient.

13. Système selon l'une quelconque des revendications précédentes, pour extraire sélectivement un agent d'un site de collecte de fluides efférents physiologiques, et facultativement, traiter successivement de manière extracorporelle ledit agent extrait.

14. Kit de pièces comprenant un système selon l'une quelconque des revendications précédentes et des instructions pour extraire sélectivement un agent d'un site de collecte de fluides efférents physiologiques et facultativement traiter successivement de manière extracorporelle ledit agent extrait.

15. Procécé pour traiter de manière extracorporelle un agent extrait d'un site de collecte d'effluents physiologiques, ledit site étant un site de collecte de fluides vasculaires, ledit procécé comprenant l'utilisation d'un système ou d'un kit selon l'une quelconque des revendications précédentes.
